# EUROPEAN PATENT SPECIFICATION

(11) **EP 4 234 679 B1**
(45) Date of publication and mention of the grant of the patent: **08.10.2025**
(21) Application number: 23178337.4
(22) Date of filing: 09.03.2021
(51) Int. Cl.: C12M 1/00, C12M 1/04, C12M 1/12, C12M 3/00

(54) **AN ASSEMBLY**
ANORDNUNG
ENSEMBLE

(30) Priority: 09.03.2020 GB 202003403; 16.12.2020 GB 202019859
(43) Date of publication of application: 30.08.2023
(62) Divisional of application: 21713069.9
(73) Proprietor: Oribiotech Ltd, London E14 9XL (GB)
(72) Inventor: VERAITCH, Farlan, London, E14 9XL (GB); RAIMES, William, London, E14 9XL (GB); PALMER, Jason, Melbourn, SG8 6DP (GB); LITTEN, Neil, Melbourn, SG8 6DP (GB); SAWFORD, Michael, Melbourn, SG8 6DP (GB); GOWLER, Vincent, Melbourn, SG8 6DP (GB)
(74) Representative: HGF

(56) References cited:
- WO-A1-2018/087558
- US-A1- 2011 076 756
- US-A1- 2011 313 375
- US-A1- 2015 368 602

## Description

### TECHNICAL FIELD OF THE INVENTION

The invention relates to an assembly for handling biological material. The invention also relates to a method of introducing material into, or removing material from, a component of such an assembly. More specifically, the invention relates to an assembly, and a method of operation thereof, for use in one or more one or more unit operations in a cell processing method, for example, in cell and/or gene therapy manufacturing processing.

### BACKGROUND

Biological handling processes, such as cell and gene therapy (CGT) manufacturing processes, are often complex and include manual steps across several devices. Equipment systems used in various steps or unit operations, of cell-based therapeutic products (CTP) manufacturing may include devices for various unit operations. The unit operations may include, for example, cell collection, cell isolation, selection, cell expansion, cell washing, volume reduction, cell storage or transportation. The unit operations can vary immensely based on the manufacturing model (i.e. autologous versus allogenic), cell type, intended purpose, among other factors. In addition, cells are "living" entities sensitive to even the simplest manipulations (such as differences in a cell transferring procedure). The role of cell manufacturing equipment in ensuring scalability and reproducibility is an important factor for cell and gene therapy manufacturing.

In addition, cell-based therapeutic products (CTP) have gained significant momentum thus there is a need for improved cell manufacturing equipment for various cell manufacturing procedures, for example but not limited to stem cell enrichment, generation of chimeric antigen receptor (CAR) T cells, and various cell manufacturing processes such as collection, purification, gene modification, incubation/recovery, washing, infusion into patient and/or freezing.

The culture or processing of cells typically requires the use of a device to hold the cells, for example, in an appropriate culture medium when culturing the cells. The known devices include shaker flasks, roller bottles, T-flasks and bags. Such bottles or flasks are widely used but suffer from several drawbacks. Chief among the problems are the requirement for transfer of cells, media or other material without contamination when passaging or processing.

A current problem in the production of cells or gene therapies for use in medicine is the absence of compact, automated closed systems for performing unit operations without contamination. For example, during cell culture, upstream or subsequent processing of cells, there is a risk of contamination when making additions to the culture vessel, or when removing cells or removing liquid samples. Moreover, the current operating systems are largely manual and hence expensive to operate. Multiple pieces of equipment are typically required to cover all of the non-cell culture steps, which involves many transfers, each of which is an opportunity for operator errors and contamination to occur.

Furthermore, with increasing manual operations comes increasing risk of manual errors and therefore the current labour-intensive processes may lack the robustness required for the manufacture of clinical-grade therapeutics. WO 2018/087558vA1 discloses a device for use in growing, culturing and/or modifying cells, the device comprising a primary container having a base section, a top section arranged substantially in parallel with the base section and a flexible wall element arranged between the top section and the base section and defining an internal lumen of the primary container, in which the wall element of the primary container is compressible with respect to the top and base section, and in which the primary container has at least one optionally sealable inlet; and wherein the device further comprises a plurality of auxiliary containers detachably connected and in fluid communication with the primary container.

Therefore, there is a need for assemblies for handling biological material, such as multistep biological material, or cell, processors, that permit such processing in an automated or, at least, in a semi-automated manner, i.e. with minimal user intervention and/or operation, whilst maintaining sterility of the overall system.

Therefore, it is an object of the present invention to provide improved assemblies for handling biological material, particularly those for use in cell and/or gene therapy processing, and more specifically an apparatus which allows for the introduction and removal of material to or from a container in a sterile and automated or semi-automated manner.

It is also an object of the present invention to provide an apparatus which combines the advantages of the cell culture containers that avoid the need for pumps and the requirement for constant passaging of cells into fresh culture devices, holding vessels, tubes etc., with the advantages conferred by having individually configurable cell and/or gene therapy processing devices.

It is a further object of the present invention to provide an apparatus that permits a variety of biological processes, such as one or more unit operations in cell processing, to be performed within a single device or apparatus having a smaller footprint and being less complex than existing equipment. Furthermore, the apparatus described herein allows for greater compatibility with automated systems. Other advantages will be apparent from the drawings and the description below.

### SUMMARY OF INVENTION

In accordance with one aspect of the present invention, there is provided an assembly for handling biological material according to claim 1 and a method of handling biological material according to claim 11.

There is provided an assembly for handling biological material, comprising:
a first biological handling element, comprising a first volume having a first port;
a second biological handling element, comprising a second volume having a second port;
an aseptic connecting element, configured to aseptically fluidly connect the first port and the second port to provide aseptic fluid communication between the first volume and the second volume;
an aseptic disconnecting element, configured to aseptically fluidly disconnect the first port and the second port to prevent aseptic fluid communication between the first volume and the second volume;
and one or more robotic arms;
wherein the one or more robotic arms are arranged to move said first biological handling element and/or said second biological handling element to bring the first port and the second port into registration with one another to allow, when in use, the aseptic connecting element to aseptically fluidly connect the first port and the second port, and to allow, when in use, the aseptic disconnecting element to aseptically fluidly disconnect the first port and the second port.

This provides the advantage that biological material may be handled in a "just-in-time" process. That is, transfer of material between volumes is provided for at the time at which transfer is required, rather than having several pre-connected components. This ensures that biological material can be handled efficiently, aseptically and in an automated, or semi-automated, manner.

In some embodiments, the first biological handling element comprises a substantially planar interface as described herein.

In some embodiments, the first biological handling element comprises a first retaining element retaining a first container having a first volume and a first port.

In particular embodiments, the substantially planar interface includes at least one port therein fluidly coupled to a container, such as a bioreactor, the container including the first volume. The substantially planar interface may include a plurality of ports. Each port of the plurality of ports may be fluidly coupled to the container. The container may comprise a top section, a base section, and a flexible or compressible wall element between the top section and the base section. The wall element may comprise a plurality of flutes or convolutes, or a bellows wall element.

In some embodiments, the second biological handling element comprises a component retaining element as described herein. The component retaining element as described herein may retain a container having the second volume and the second port.

In some embodiments, the second biological handling element comprises a second retaining element retaining a second container having a second volume and a second port.

In particular embodiments, the component retaining element is configured to retain, or retains, a component. The component may comprise a container, having the second volume, and comprising the second port. The container may comprise a top section, a base section, and a flexible or compressible wall element between the top section and the base section. The wall element may comprise a plurality of flutes, or a bellows wall element. The container may be a bag, a flexible bag, or a container comprising a plunger.

The first port and/or the second port may comprise an open-ended tube, a closed-ended tube, or a tube having a removable aseptic membrane disposed over an end thereof. The tube may be a flexible tube. Alternatively, or in combination, the first port and/or the second port may comprise a hermetic seal, a septum seal, a Luer lock port or the like.

The aseptic connecting element may be unitary with the aseptic disconnecting element. That is, there may be provided a unitary aseptic connecting and disconnecting element. The unitary aseptic connecting and disconnecting element may comprise a connector, such as a sterile or aseptic connector as described herein. In particular, the connector may comprise a needle. The needle may be actuatable to engage or pierce the first port and the second port, such as a first septum seal and a second septum seal, during use.

The aseptic connecting element may be a separate component with respect to the aseptic disconnecting element.

The aseptic connecting element may comprise an aseptic welding element. In particular, the aseptic connecting element may comprise an aseptic tube welder, configured and/or arranged to aseptically weld a first tube, such as the first port, to a second tube, such as the second port. The aseptic connecting element may comprise a robotic arm terminating in an aseptic welding element. The aseptic welding element may be configured to apply heat, and optionally pressure, to the respective ports so as to heat weld the same. The aseptic welding element may also be configured to apply heat so that the closed-end of a tube, or the aseptic membrane covering the end of a tube, is melted thereby enabling a fluid passageway through the respective tube.

The aseptic disconnecting element may comprise an aseptic sealing element. In particular, the aseptic disconnecting element may comprise an aseptic tube sealer, configured and/or arranged to aseptically seal a first portion of a tube, such as the first tube or the first port, with respect to a second port of a tube, such as the second tube or the second port. The aseptic disconnecting element may comprise a robotic arm terminating in an aseptic sealing element. The aseptic sealing element may be configured to apply heat, and optionally pressure, to a portion of the connected ports so as to heat seal the same, thereby providing a first portion of the connected ports heat sealed from a second portion of the connected ports. The aseptic sealing element may be further configured to cut, or otherwise separate, the sealed region between the first portion and the second portion of the connected ports, thereby allowing release of the respective components.

The apparatus may comprise a component retaining element as described herein. The component retaining element may be configured to retain the first biological handling element and/or the second biological handling element.

The apparatus comprises one or more robotic arms. The one or more robotic arms are arranged to move the first biological handling element and/or the second biological handling element to bring the first port and the second port into registration with one another. The one or more robotic arms may comprise a retaining portion arranged to retain the first biological handling element and/or the second biological handling element. The one or more robotic arms may comprise an actuator portion arranged to actuate a portion of the first biological handling element and/or the second biological handling element to cause dispensation of biological material from the first volume and/or the second volume. The one or more robotic arms, the retaining portion and/or the actuator portion may be controlled by a controller. The controller may comprise a user interface. The controller may be arranged to control the one or more robotic arms, the retaining portion and/or the actuator portion in response to user input to the user interface.

The apparatus may be an automated apparatus. That is, the apparatus does not require manual or user intervention. The apparatus may be a semi-automated apparatus. That is, the apparatus may require minimal manual or user intervention, such as the loading of biological handling elements into the apparatus.

The apparatus may comprise an incubator or a housing enclosing the respective components of the apparatus.

In accordance with yet another aspect of the present invention, there is provided a method of handling biological material, comprising:
moving, using one or more robotic arms, at least one of a first biological handling element, comprising a first volume having a first port, and a second biological handling element, comprising a second volume having a second port, to bring the first port and the second port into registration with one another ;
aseptically fluidly connecting the first port and the second port, thereby providing fluid communication between the first volume and the second volume;
transferring biological material from the first volume to the second volume, or from the second volume to the first volume; and
aseptically fluidly disconnecting the aseptically fluidly connected first port and second port, thereby preventing fluid communication between the first volume and the second volume.

This provides the advantage that biological material may be handled in a "just-in-time" process. That is, transfer of material between volumes is provided for at the time at which transfer is required, rather than having several pre-connected components. This ensures that biological material can be handled efficiently, aseptically and in an automated, or semi-automated, manner.

In certain embodiments, the step of aseptically fluidly connecting the first port and the second port comprises aseptically welding the first port to the second port. In particular embodiments, the step of aseptically welding may be carried out by an aseptic tube welder.

In certain embodiments, the step of aseptically fluidly disconnecting the first port and the second port comprises aseptically sealing the connected first port the second port to provide an aseptically sealed first port and an aseptically sealed second port. In particular embodiments, the step of aseptically sealing may be carried out by an aseptic tube sealer.

It will be appreciated by those skilled in the art that the method may include one or more components of the apparatus as described above or elsewhere herein, such as the substantially planar interface, the component retaining element, the aseptic tube welder, the aseptic tube sealer, or the like.

### BRIEF DESCRIPTION OF THE DRAWINGS

Example embodiments of the invention are illustrated with figures 24(a) to 24 (d). Figures 1 to 23 are reference examples.
**Figure 1** illustrates a perspective overview of a system including an assembly in accordance with the invention, an enclosure and a component;
**Figure 2** illustrates a perspective view of the assembly, the enclosure and the component of Figure 1, during use;
**Figure 3** illustrates a perspective view of a planar interface, including a container attached thereto;
**Figure 4** illustrates a cross-sectional side view of the assembly of Figure 3, including an expandable receptacle and a cover attached to the planar interface;
**Figure 5** illustrates a perspective view of another planar interface, including a container attached thereto;
**Figure 6** illustrates (a) a perspective view of another planar interface, including a container attached thereto, (b) a cross-sectional view of the planar interface of (a), (c) an enlarged cross-sectional view of the planar interface of (a), and (d) another cross-sectional view of the planar interface of (a);
**Figure 7** illustrates a perspective view of the assembly of Figure 5, and further including a component retaining element of an assembly;
**Figure 8** illustrates (a) a perspective view of loading the planar interface of Figure 5 into a drawer of an enclosure, (b) a perspective view of the planar interface loaded into the drawer shown in an open position, and (c) a perspective view of the enclosure with the drawer in the closed position;
**Figure 9** illustrates (a) an enlarged perspective view of Figure 8(b), (b) a partially cut-away perspective view of Figure 8(c), and (c) an enlarged perspective view of (b) illustrating a drive mechanism;
**Figure 10** illustrates (a) a perspective view of another enclosure, illustrating locating features of another planar interface and the drawer of the enclosure, and (b) a perspective view of (a) illustrating the planar interface loaded into the drawer;
**Figure 11** illustrates a partially cut-away front view of the enclosure of Figure 8(c) including a component retaining element formed as part of the enclosure;
**Figure 12** illustrates (a) a cross-sectional side view and of a component and (b) a cross-sectional perspective view of a connector of the component of (a);
**Figure 13** illustrates (a) a partially cut-away perspective view of Figure 8(c), having the component retaining element omitted, (b) a cross-sectional perspective view of (a), (c) another perspective view of (a) illustrating an agitation mechanism and an agitation mode thereof, and (d) a side view of (a) illustrating another agitation mode of the agitation mechanism illustrated in (c);
**Figure 14** illustrates (a) a perspective view of another enclosure, (b) a perspective view of loading the planar interface of Figure 5 into the enclosure of (a), and (c) an enlarged view of (b);
**Figure 15** illustrates a perspective view of the enclosure of Figure 14(a), having (a) an upper access door in a closed position, and (b) an access door in an open position;
**Figure 16** illustrates (a) a perspective view of a component and the enclosure of Figure 14(a) prior to loading, (b) an enlarged perspective view of Figure 15(b) illustrating the component being loaded to a component retaining element of the assembly, and (c) an enlarged perspective view of Figure 15(b) illustrating a component loaded to a component retaining element of the assembly;
**Figure 17** illustrates a perspective view of (a) the component retained by the component retaining element as shown in Figure 16(c), (b) initial actuation of the component by the component retaining element, and (c) further actuation of the component by the component retaining element to bring the component into engagement with the planar interface;
**Figure 18** illustrates a schematic side view of (a) the component retained by the component retaining element as shown in Figure 16(c), (b) engagement of the component to the planar interface such that they are in registration with one another, and (c) removal of aseptic barriers of the component and the planar interface to bring the respective ports into engagement with one another;
**Figure 19** illustrates a perspective view of an aseptic barrier removal system and particularly (a) a first step, (b) a second step, and (c) a third step of actuating the aseptic barrier removal system;
**Figure 20** illustrates a perspective view of the component, including a connector, retained by the component retaining element as shown in Figure 17(c), (a) prior to actuation of the connector, and (b) after actuation of the connector;
**Figure 21** illustrates a cross-sectional side of the connector of Figure 20 during various steps of actuation of the same, including (a) prior to actuation of the connector, (b) after initial actuation of the connector, (c) after further actuation of the connector, and (d) cessation of actuation of the connector;
**Figure 22** illustrates a perspective view of the component retained by the component retaining element as shown in Figure 17(c), further including a dispensing actuator, (a) prior to dispensing, and (b) after dispensing;
**Figure 23** illustrates a schematic side view of the component retained by the component retaining element as shown in Figure 17(c) removed from the planar interface; and
**Figures 24(a) to 24(d)** illustrate a method of aseptically connecting and disconnecting biological handling components according to one example.

### DETAILED DESCRIPTION

The described example embodiments relate to an assembly for handling biological material. In particular, some embodiments relate to an assembly that is aseptic, or sterile. It is noted that the terms "aseptic" and "sterile" may be used interchangeably throughout the present disclosure. References to fluids in the detailed description are not intended to limit the scope of protection to such materials. As will be recognised by a person skilled in the art, fluids as described herein are merely an example of a suitable material for use with the assembly as described. Equally, reference may be made to a container, receptacle, or the like, however, such references are not intended to limit the scope of protection to such containers or receptacles. As will be recognised by a person skilled in the art, containers, receptacles or the like are described herein as mere examples.

Certain terminology is used in the following description for convenience only and is not limiting. The words 'upper' and 'lower' designate directions in the drawings to which reference is made and are with respect to the described component when assembled and mounted. The words 'inner', 'inwardly' and 'outer', and 'outwardly' refer to directions toward and away from, respectively, a designated centreline or a geometric centre of an element being described (e.g. a central axis), the particular meaning being readily apparent from the context of the description. Further, the terms 'proximal' (i.e. nearer to) and 'distal' (i.e. away from) designate positions relative to an axis or a point of attachment.

Further, as used herein, the terms 'connected', 'affixed', 'coupled' and the like are intended to include direct connections between two members without any other members interposed therebetween, as well as, indirect connections between members in which one or more other members are interposed therebetween. The terminology includes the words specifically mentioned above, derivatives thereof, and words of similar import.

Further, unless otherwise specified, the use of ordinal adjectives, such as, 'first', 'second', 'third' etc. merely indicate that different instances of like objects are being referred to and are not intended to imply that the objects so described must be in a given sequence, either temporally, spatially, in ranking or in any other manner. Like reference numerals are used to depict like features throughout.

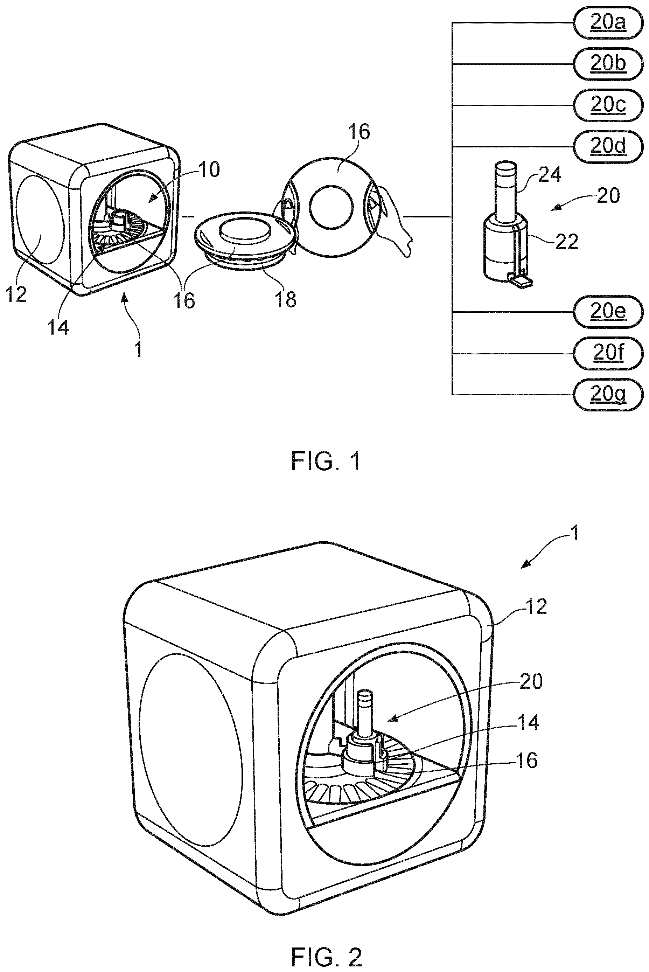

As shown in the reference example in Figure 1, a biological material handling system 1 is illustrated including an assembly 10, for handling biological material.

In particular, the system 1 includes an enclosure 12, a component retaining element 14, a planar interface 16, a container 18 and a component 20. The component 20 in this particular example is a connector 22 including a receptacle 24 fluidly attached thereto. The component 20 may be another component, such as an addition component, for example, one of a cell loading component 20a, for loading cells into the container 18; a bead loading component 20b, for loading beads, such as magnetic beads, into the container 18; a virus loading component 20c, for loading virus into the container 18; or a media loading component 20d, for loading media, such as biological media suitable for cell residence, into the container 18. Furthermore, the component 20 may be a removal component, for example, one of a cell removal component 20e, for removing cells from the container 18 into a suitable container; a sampling component 20f, for withdrawing a sample of the contents of the container 18; or a media removal component 20g, for removing a portion, most or all of the media or contents of the container 18. Generally, in the described embodiments, the component 20 includes a connector 22 and a receptacle 24, the receptacle 24 being arranged for a suitable use as an addition or removal component 20a-20g.

Referring further to Figure 2, the enclosure 12 forms an interior volume for housing the component retaining element 14, the planar interface 16, the container 18 and the component 20 therein. The enclosure 12 generally assumes the form of an incubator unit. The component retaining element 14 is provided integrally with the enclosure 12, i.e. the component retaining element 14 is formed as part of the enclosure 12 in an interior portion thereof, and is arranged to hold, receive or otherwise retain the component 20 during use. Furthermore, as shown in Figure 1, the planar interface 16 is coupled to a container 18, such as a bioreactor which may include flexible walls or be otherwise collapsible, so as to be fluidly and aseptically connected thereto. In use, the planar interface 16 and the container 18 are housed within the enclosure 12 as shown in Figures 1 and 2.

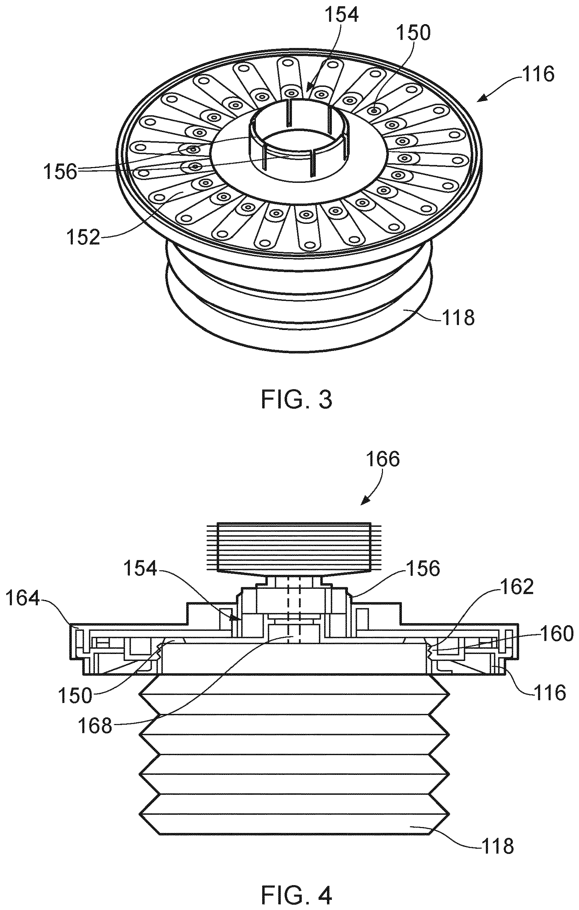

Figure 3 illustrates a portion of a reference assembly. In particular, Figure 3 illustrates a planar interface 116 having a container 118, such as a bioreactor, mounted thereto. In particular, the planar interface 116 includes a screw thread, as discussed in relation to Figure 4 below, for coupling to a complementary screw thread of the container 118. Thus, an aseptic and fluid-tight, or hermetic, seal is formed between the planar interface 116 and the container 118. The planar interface 116 includes a plurality of ports 150, formed as twenty-two septum seals in the present example, which allow a needle to pierce therethrough and thus allow handling of biological material or fluid. Any number of septum seals may be used. Such uses may include, for example, sampling contents of the container 118 in use, additions to the container 118 in use and/or removing contents, i.e. wasting, from the container 118 in use. Generally, each port 150 is arranged to provide a fluid passageway, in use and as described below, through the planar interface 116 to the container 118. The person skilled in the art would recognise that other ports may also be used, having openings, doors, valves or the like so as to enable selected fluid communication therethrough.

As shown in this example, each individual port 150 has an aseptic barrier 152 disposed thereover. The aseptic barrier 152 is generally removable attached to each port 150 at one surface thereof. The aseptic barrier 152 ensures sterility of each port 150 prior to use, and is generally removed prior to making a connection to each port 150. The aseptic barrier 152 generally assumes the form of an aseptic skin, composed from a paper or a polymer material, in the present example. Each aseptic barrier 152 is arranged to be removed by a portion, such as a barrier removal system, of the enclosure 12 (see enclosure of Figure 1) or by the user, as described further below.

The planar interface 116 of the present example further includes a central hub 154, upstanding from an upper surface of the planar interface 116, and including a plurality of coupling elements 156, formed as clips in the present embodiment, which are arranged to receive and couple to an expandable receptacle, as described in relation to Figure 4.

Further, the container 118 in the present example, formed as a bioreactor having an internal chamber, a base wall and a compressible side wall, has a maximum volume of 1.5L. Generally, the base of the container 118 , i.e. the cell growing area, may be 150sq cm. Additionally, in some embodiments not shown, the container 118 may accommodate a 0.2 micron filter, formed as part of the base or the side walls of the container 118 or, alternatively, formed as part of the planar interface 116. The filter may provide gaseous exchange between the surrounding environment, such as the enclosure, and the internal chamber of the container 118. The container 118 may generally be manufactured using a blow moulding method, such as extrusion or injection blow moulding, which provides a simplified manufacture of a container 118, having a single piece container, i.e. no joints and so no leak paths, a flatter base, and also provides less plastic for entering the clinical waste stream. The container 118 may be composed of any suitable material, such as low density polyethylene (LDPE), high density polyethylene (HDPE), a thermoplastic elastomer (TPE) or silicon.

Figure 4 shows the portion of the assembly of Figure 3, including the planar interface 116 and the container 118. As illustrated, the planar interface 116 couples to the container 118 by virtue of a threaded portion 160 of the planar interface 116 which is complementary to a threaded portion 162 of the container 118. Furthermore, in the present example, a cover 164 is provided disposed over an upper surface of the planar interface 116, and coupled, for example clipped, thereto, which may be removed prior to use, or retained during use. The central hub 154 of the planar interface 116 is shown as coupled to an expandable receptacle 166, shown in the compressed configuration in Figure 4. The expandable receptacle 166 includes coupling elements, such as clips, that are complementary, and couple, to the coupling portions 156 of the central hub 154. In one particular example, the expandable receptacle 166 includes a screw thread that is complementary with a screw thread formed in the central hub 154. The expandable receptacle 166 is fluidly coupled to the container 118 by virtue of a fluid passageway 168, indicated by dashed lines. The fluid passageway 168 may be formed by a port (not shown) in the planar interface 116, or an aperture (not shown) in the planar interface 116, which communicates with a respective port or aperture (not shown) of the expandable receptacle 166. In this way, a breathing mechanism is provided, in which fluid, such as air, from the container 118 can enter the expandable receptacle 166, upon compression of the container 118, thereby causing expansion of the expandable receptacle 166 into an expanded configuration. Likewise, during use, fluid such as air from the expandable receptacle 166 can be drawn into the container 118 upon expansion thereof, thereby causing compression of the expandable receptacle 166 into a compressed configuration. Thus, the pressure within the container 118 is maintained at a substantially constant level during compression and decompression of the same. In other embodiments, not shown, the expandable receptacle 166 may be replaced by a filter, which provides the breathing mechanism by allowing fluid, such as air, to escape the container 118 or drawn into the container 118 as required. Such a filter may be gas-permeable, or oxygen-permeable and/or carbon-dioxide-permeable, and liquid-impermeable.

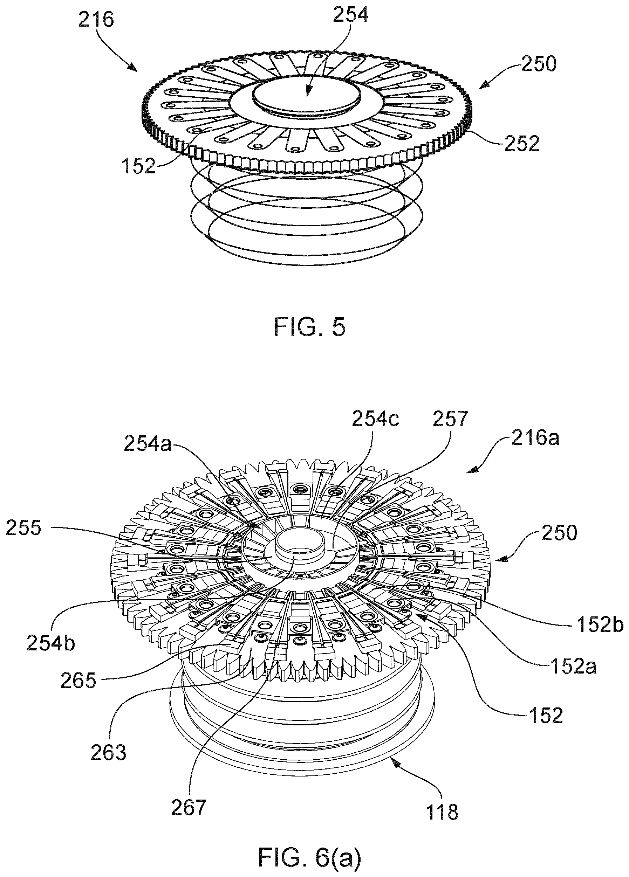

Figure 5 illustrates another planar interface 216 as described further below. The planar interface 216 is substantially the same as planar interface 116 of Figures 3 and 4 in that it includes a number of ports (not shown) having aseptic barriers 152 disposed removably attached thereto and thereover. The planar interface 216 further includes a circumferential rim 250 having a number of drive elements 252, formed as a series of grooved portions in the present example, arranged to cooperate with a drive mechanism of the enclosure, as described further below. The planar interface 216 also includes a central hub 254 upstanding from the upper surface of the planar interface 216, which is shown as not being connected to an expandable receptacle, but may have features that allow connection to an expandable receptacle or filter, such as those as described in relation to Figures 3 and 4.

Figures 6(a) to 6(c) illustrate yet another planar interface 216a as described further below. The planar interface 216a is substantially the same as the planar interfaces 116, 216 of Figures 4 and 5, and so like features, such as the circumferential rim 250, and the container 118, will not be described further.

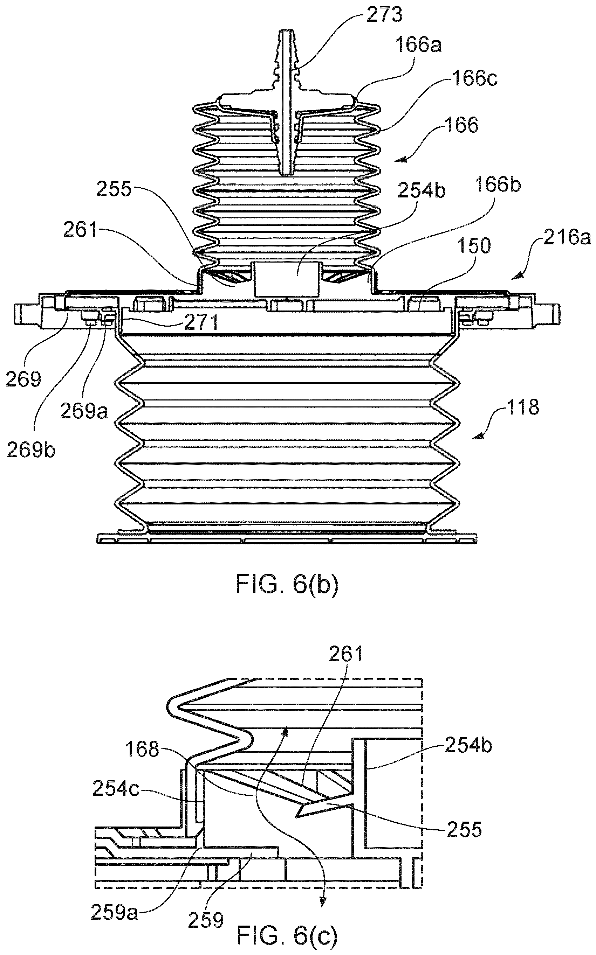

The planar interface 216a includes a central hub 254a having an inner circular wall 254b and an outer, concentrically arranged, circular wall 254c. The outer circular wall 254c is arrange to allow the coupling of an expandable receptacle 166, as shown in Figure 6(b), during use. For example, the outermost surface of the outer circular wall 254c may include a screw thread, a clip portion or the like, to enable coupling of the expandable receptacle 166.

The inner circular wall 254b includes a radially extending flange 255 extending from the inner circular wall 254b radially outwardly towards the outer circular wall 254c. The flange 255 is illustrated as being C-shaped, although the flange 255 may extend around the entire circumference of the inner circular wall 254b. In this example, the central hub 254a is further provided with a dividing wall 257 that extends from, and connects, a portion of the outer circular wall 254c to a portion of the inner circular wall 254b, for example, the portion of the inner and outer circular walls 254b, 254c which does not include the C-shaped flange 255. As such, the dividing wall 257 may connect opposing ends of the C-shaped flange 255.

The outer circular wall 254c includes a ledge 259 extending from a base 259a thereof radially inwardly towards the inner circular wall 254b. Generally, the ledge 259 and the flange 255 define a fluid passageway 168 therebetween, particularly a fluid passageway 168 between the lower surface of the planar interface 216a, to which a container 118 is attached, and the upper surface of the planar interface 216a, particularly the central hub 254a, to which an expandable receptacle 166 is attached, as illustrated best in Figure 6(b) and 6(c). In this way, the central hub 254a defines a fluid passageway 168 from an attached container 118 to an attached expandable receptacle 166 to allow breathing, or compensation of air pressure, between the respective containers 118, 166. More specifically, the ledge 259 and the flange 255 define a tortuous, or non-linear, fluid passageway 168, so that air or gases can pass through the passageway 168, whilst inhibiting fluid, such as media including biological material, from passing through such passageway 168 and into the expandable receptacle 166.

There is further provided a series of radially extending ribs 261, connecting the flange 255 to the inner surface of the outer circular wall 254c, each rib 261 being spaced apart from one another. Each adjacent pair of ribs 261 defines an individual fluid passageway 168 therebetween. Furthermore, as illustrated best in Figure 6(c), the flange 255 forms an angle to the horizontal, namely the horizontal plane in which it is located, so that the flange 255 is orientated, or angled, downwardly, or towards the ledge 259 of the outer circular wall 254c. In this way, any liquid or condensate arising from the container 118 is directed back towards the container 118, so that material is not lost from the container 118 into the expandable receptacle 166, during use.

Furthermore, the planar interface 216a includes a number of ports 150 (see Figure 6(b)) having a removable aseptic barriers 152 disposed removably attached thereto and thereover so as to aseptically seal the same. In particular, the removeable aseptic barriers 152 include a fastener portion 152a coupled to an aseptic skin 152b, the aseptic skin 152b being aseptically coupled to each port 150 (see Figure 6(b)). The fastener portion 152a is disposed within a space 263 formed between respective guide walls 265 and retained in place by means of a clip 267 attached to adjacent guide walls 265. Thus, the fastener portion 152a is slidable within the space 263 so that the aseptic skin 152b can be removed from the port 150 during use and as described in further detail below.

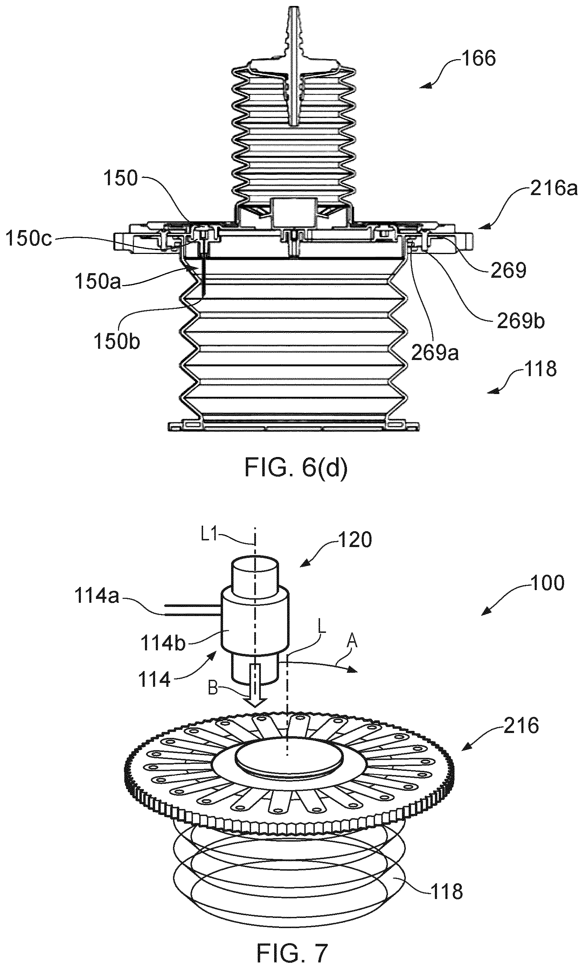

With particular reference to Figure 6(d), the planar interface 216a further includes a low volume sampling element 150a extending from a lower surface of the planar interface 216a. More particularly, the low volume sampling element 150a is formed integrally with one or more of the ports of the planar interface 216a. The low volume sampling element 150a is formed as a hollow tubular member 150b, including a through bore extending from a distal end thereof to a proximal end thereof in communication with the respective port 150, and comprises a resilient material, such as a thermoplastic elastomer or silicone. In particular, the hollow tubular member 150b is coupled to the lower surface of the planar interface 216a, adjacent the port 150, by means of a screw cap 150c, which includes a screw thread that threadingly engages a corresponding screw thread of the hollow tubular member 150b. The screw cap 150c may be coupled to the lower surface of the planar interface 216a by any appropriate means, such as an adhesive, a clip or the like.

During use, the container 118 and/or the planar interface 216a may be moved so that the hollow tubular member 150b extends into a liquid held within the container 118 to enable sampling of the same. Additionally, due to the resilient nature of the hollow tubular member 150b, the same may be arranged to contact the base of the container 118, without damaging the same. In particular, the hollow tubular member 150b, being of resilient material, is arranged to bend into, or towards, a corner of the container 118, particularly a corner adjoining the base of the container 118 to a side wall of the container 118. As such, samples may be taken from the container 118, even at low volumes, without risking damage to the container 118. More particularly, in this specific example, a needle of a connector, as discussed below, is inserted through the port 150 so that the throughbore thereof is in fluid communication with the throughbore of the hollow tubular member 150b. A component coupled to the other end of the needle of the connector may then be actuated so as to draw fluid from the container 118, through the hollow tubular member 150b, through the needle of the connector and into or towards the component. As such, a sample may be taken from the container 118.

Furthermore, as illustrated in Figures 6(b) and 6(d), the container 118 in this example is coupled to the planar interface 216a by means of a clamp ring 269. The clamp ring 269 includes a protrusion 269a that frictionally engages the outer surface of the container 118 and clamps the container 118 between the protrusion 269a of the clamp ring 269 and a coupling wall 271 extending distally from the lower surface of the planar interface 216a. The clamp ring 269 is coupled to the planar interface 216a by way of a plurality of fasteners 269b, such as screws or bolts. Alternatively, the container 118 may include a screw thread which engages a screw thread of the planar interface 216a, thereby negating the need for a clamp ring 269.

Furthermore, as shown in Figure 6(b) and 6(d), there is provided an expandable receptacle 166 coupled to the central hub 254a. The expandable receptacle 166 includes a top section 166a, a base section 166b and a wall section 166c extending between the base and top sections 166a, 166b. The wall section 166c is formed as a bellows with a plurality of foldable convolutes, thereby allowing expansion and compression of the receptacle 166. The top section 166a of the expandable receptacle 166 comprises an air filter 273, communicating with the internal volume of the expandable receptacle 166 and the external environment. The air filter 273 is arranged to allow air to pass into, or out of, the expandable receptacle 166, without allowing liquids to pass into, or out of, the same. The air filter 273 may be gas-permeable, such as oxygen or carbon dioxide permeable, and liquid-impermeable. Generally, the air filter 273 is an aseptic air filter, and as such may filter bacteria, viruses or other material which would otherwise cause an aseptic environment within the container 118 and/or the expandable receptacle 166. The base section 166b of the expandable is open-ended and includes a circular base wall 166d arranged to couple to the central hub 254a of the planar interface 216a. In particular, the circular base wall 166d is coupled to the outer circular wall 254c of the central hub 254a by means of a spring clamp or spring clip (not shown). Alternatively, the circular base wall 166d may include an external thread configured to engage a thread on the outer circular wall 254c of the central hub 254a.

Figure 7 illustrates an assembly 100 including the planar interface 216 of Figure 5, and a container 118. Alternatively, the planar interface 116 of Figure 3, 4 or 6(a) may be used. The assembly includes a component receiving element 114, such as the component receiving element 14 formed as part of the enclosure 12 of Figures 1 and 2, for holding a component 120, such as the component 20 of Figures 1 and 2. In particular, the component receiving element 114 is formed by an arm 114a, extending from an interior of the enclosure (not shown), and terminating in a component retaining head 114b. The component retaining head 114b includes a retaining element for retaining, receiving or otherwise holding the component 120 in use. For example, the component retaining head 114b may include a rail for receiving a portion of the component 120 so as to allow the component 120 to slide within the component retaining head 114b as required. In other examples, the component retaining head 114b retains the component 120 by virtue of a friction fit. In further examples, the component retaining head 114b includes a base and one or more sides walls, thereby forming a volume into which the component 120 is received, particularly the component 120 resting or bearing against the base, during use. As will be recognised by the person skilled in the art, any appropriate mechanism of retention of the component 120 to the component retaining element 114 is contemplated.

Generally, the component retaining element 114 and/or the planar interface 216 are moveable so as to align, or bring into register, a port of the component 120 with one of the ports of the planar interface 216. In this particular example, as illustrated by arrow A, the planar interface 216 is rotatable about a central longitudinal axis L of the planar interface 216. Additionally, the component retaining element 114 is substantially immovable laterally, i.e. within the plane formed by the component retaining element 114, but longitudinally moveable along a longitudinal axis L1 of the component retaining element 114, specifically the component retaining head 114b, such as in the direction indicated by arrow B. Thus, during use and as described further below, the planar interface 216 is caused to rotate, about central longitudinal axis L, such that a port of the component 120 held by the component retaining head 114b of the component retaining element 114 aligns with a port of the planar interface 216, and then the component retaining head 114b, or alternatively the entirety of the component retaining element 114, is caused to move along the axis L1, in direction B, to bring the port of the component 120 into engagement with the port of the planar interface 216, particularly the aseptic barriers 152 in the first instance. Thereafter, the component retaining head 114b, or the component retaining element 114 in its entirety, is caused to move along the axis L1, in an opposing direction to direction B, to remove the engagement between the respective ports. Thereafter, the planar interface 216 is caused to rotate, about central longitudinal axis L, to align the next port of the planar interface 216 with a port of the component 120 in a sequential manner. The process may then be repeated any number of times. The detailed operation of the assembly 100 is described below.

Generally, the assembly 100 described provides an assembly that is more suitable to automation, and requires less manual intervention or operation. In other examples, the arm may be rotatable and moveable so as to align the respective ports, and the planar interface may be substantially immoveable. Further examples may also include both a moveable arm and a moveable planar interface.

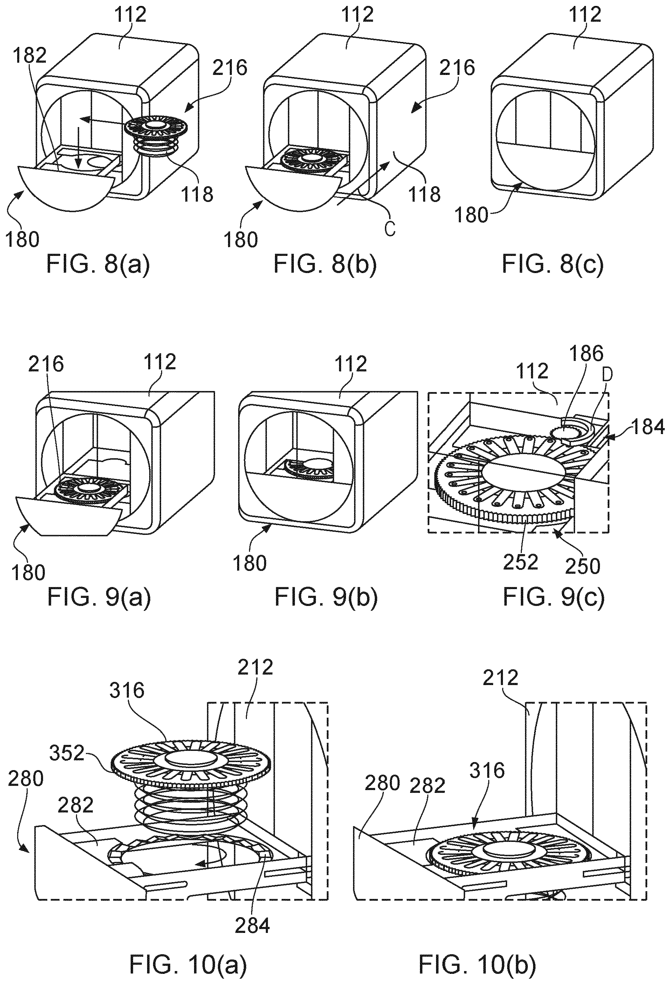

Figures 8(a) to 8(c) illustrate a method of loading the planar interface 216 and container 118 of Figure 7 into an enclosure 112. As shown in Figure 8(a), the enclosure 112 includes a sliding drawer 180, having a loading plate 182, slidable on telescopic rails between an open configuration (see Figures 8(a) and (b)), in which the planar interface 216 may be loaded into the drawer 180, and a closed configuration (see Figure 8(c)), in which the drawer 180 is located and housed within the enclosure 112. Generally, in use, a user slides the drawer 180 open into an open configuration, in a direction perpendicular to the central longitudinal axis of the planar interface 216, and loads the planar interface 216 onto the loading plate 182 and thus into the drawer 180 (Figure 8(a)). Once loaded, the user slides the drawer 180, and thus the planar interface 216, closed, in direction C indicated, into the closed configuration (Figure 8(b)). In the closed configuration, the planar interface 216 and receptacle 118 are loaded into, and housed within, the enclosure 112, particularly the interior volume thereof (Figure 8(c)), and use of the system may begin.

Figure 9(a) to 9(c) illustrates a more detailed view of the enclosure 112, the planar interface 216 and a drive mechanism 184 of the enclosure 112. As shown in Figures 9(a) and 9(b), the planar interface 216 is loaded into the drawer 180 of the enclosure 112 as described above. Once loaded, and as shown in Figure 9(c), a drive mechanism 184, particularly a grooved drive wheel 186, engages with the circumferential rim 250 having the plurality of drive elements 252. The grooved drive wheel 186 and the drive elements 252 of the circumferential rim 250 mesh during use so as to act as a gear arrangement, thereby providing rotation of the planar interface 216 about its central longitudinal axis during use. In particular, the drive wheel 186 may be driven clockwise, as shown by arrow D, by a motor thereby driving the planar interface 216 in an anticlockwise manner about its central longitudinal axis.

The drive mechanism 184, particularly the drive wheel 186, may be driven by a controller (not shown) of the enclosure 112, which may be coupled to a number of sensors (not shown) for sensing the positioning of the planar interface 216 within the enclosure 112.

To this end, the planar interface 216 may include a number of magnets and the enclosure 112 may include a Hall effect sensor, or vice versa. The controller may control the positioning of the planar interface 216, particularly with respect to the component retaining element during use, so as to position the respective ports of the planar interface and the component during use. Thus, a controlled or automated homing and/or rotation of the planar interface 216 is provided within the enclosure 112 without manual intervention or operation. In this way, the planar interface 216 may be loaded into the enclosure 112 without the user having to correctly align the same, i.e. the planar interface 216 may be placed into the enclosure without aligning to a port of the component held by the component retaining element. The planar interface 216 is then located and rotated by the controller, in accordance with a user input such as the selection of a particular port of the planar interface 216 on a user interface of the enclosure 112, controlling the drive wheel 186 so as to align the respective ports of the planar interface 216 and the component. The drive wheel 186 may, thereafter, control subsequent movement of the planar interface 216 so as to align another port of the planar interface 216 with a port of the component in a sequential manner as described below, thereby providing an automated system.

Figures 10(a) and 10(b) illustrate another planar interface 316 and another enclosure 212. The planar interface 316 is substantially the same as planar interface 216 of Figures 5, 6 and 7, except in that the planar interface 316 includes a number of locating features 352 formed on a lower surface, i.e. an underside, of the planar interface 316. The locating features 352 are formed as a sinusoidally-shaped portion, such as a cut-out portion, of the lower surface in this particular example. Moreover, the planar interface 316 does not include a circumferential rim having a grooved outer surface, as shown in the planar interface 216 of Figures 5, 6 and 7, but instead includes a smooth circumferential rim 350. In other embodiments, the planar interface 316 may include both the locating features 352 on an underside of the planar interface 316, and a groove circumferential rim.

The enclosure 212 similarly includes a drawer 280, as described in relation to Figures 8(a) to 8(c). However, in the present example, the drawer 280 includes a loading plate 282 including corresponding locating features 284 on an upper surface thereof. The locating features 284 are formed as a sinusoidally-shaped portion, such as a cut-out portion, of the upper surface in this particular example. Thus, the locating features 284 are complementary to the locating features 352 of the planar interface 316. In this way, the user is provided with tactile feedback that the planar interface 316 is seated within the drawer 280. In use, the assembly 212 may cause rotation of the planar interface 316 in a manner as described in relation to Figures 9(a) to 9(c), or, alternatively, the drawer 280, particularly the loading plate 282, may be caused to rotate by a drive mechanism, as indicated by the arrow in Figure 10(a). Thus, the locating features 284, 352 may provide confidence to a user that the planar interface 316 is seated to the drawer 280, whilst negating the need for a user to load the planar interface 316 in a specific orientation.

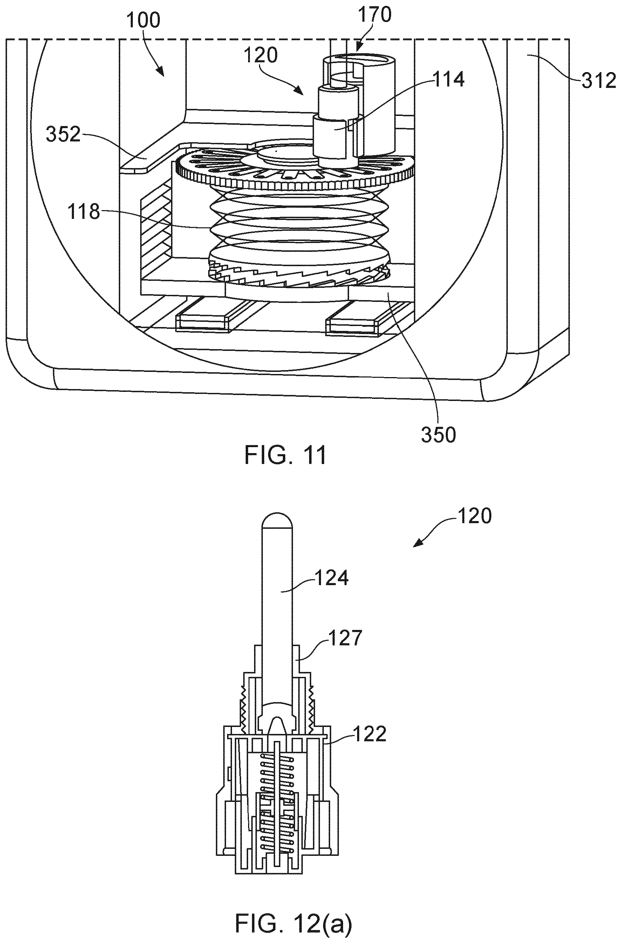

Figure 11 illustrates the assembly 100 of Figure 6 housed within an enclosure 312, which may be any one of the enclosures discussed above. In particular, as shown in Figure 11, the assembly 100 includes the component retaining element 114, formed as part of an interior portion of the enclosure 312 in the present example, retaining a component 120.

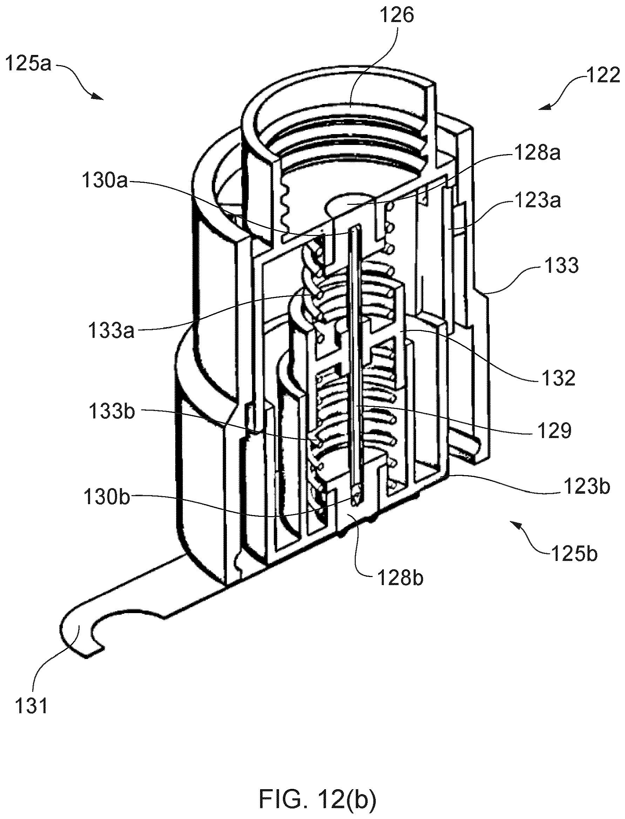

Referring further to Figure 11, the component retaining element 114 is shown as further including an actuator 170, arranged to actuate the component 120, specifically the connector 124 (see Figures 12(a) and 12(b)) thereof. For example, with reference to Figures 11, 12(a) and 12(b), the actuator 170 may cause fluid coupling between the second septum seal 128b of the connector 122 and a port of the planar interface 116, as described further below. The planar interface 116 is housed within the enclosure 312, specifically between an agitation plate 350, engageable with a base surface of the container 118 attached to the planar interface 116, and a plate 352, which may house or receive the planar interface 116 and/or include a drive mechanism (not shown) for driving the planar interface 116. The planar interface 116 may be housed within the enclosure 312 in accordance with any one of the examples discussed above, for example, by virtue of a drawer.

With further reference to Figures 12(a) and 12(b), the component 120 in this example includes a connector 122 having a receptacle 124, such as a vacutainer, attached thereto. Thus, in this particular example of component 120, a sampling component 20e (see Figure 1) is provided. As will be apparent to the person skilled in the art, this is merely one example of a component which can be used with the present assembly.

Referring to Figures 12(a) and 12(b), the connector 122 of the component 120 shall be described. The connector 122 is generally arranged to connect two volumes of fluid. The connector 122 includes a housing including an upper housing portion 123a and a lower housing portion 123b. The housing extends along a longitudinal axis between a distal end 125a and a proximal end 125b. The upper housing portion 123a is axially moveable, or slidable, with respect to the lower housing portion 123b, as will be described further below.

The housing includes a threaded portion 126 at its distal end 125a for connecting to a corresponding threaded portion of a receptacle, such as a threaded screw cap 127 of the receptacle 124 illustrated in Figure 12(a), the receptacle including a first volume of fluid. As will be clear to the skilled person, the housing may be provided without the threaded portion 126, and instead be provided with another suitable connection mechanism for connecting to a portion of a receptacle. Further, a receptacle may be directly attached to the distal end 125a by any suitable mechanism, for example, the receptacle may be pre-connected or sealed, for example, through an adhesive or the like. In some embodiments, the receptacle is manufactured comprising a connector 122.

In this embodiment, the connector 122 includes a first port, formed as a first septum seal 128a, disposed at the distal end 125a of the housing, and a second port, formed as a second septum seal 128b, disposed at the proximal end 125b of the housing. The housing further includes a hollow needle 129 that is mounted, biasedly mounted in this particular example, within the housing. The hollow needle 129 is generally coaxially aligned with the longitudinal axis of the connector 122. The hollow needle 129 includes a first end 130a, facing the first septum seal 128a, and a second end 130b, facing the second septum seal 128b. The first end 130a is configured to be able to pierce through the first septum seal 128a, in use, and the second end 130b is configured to be able to pierce through the second septum seal 128b, in use. The first septum seal 128a, the second septum seal 128b, or both the first and second septum seal 128a, 128b may optionally be provided with an aseptic barrier 131 disposed thereover. The aseptic barrier 131 is generally configured to mate with a corresponding aseptic barrier 152 (see Figure 3) of a planar interface 116, 216, 216a, 316 (see Figures 3 to 7 and 8). The aseptic barrier 131 may be arranged to adhere to, and otherwise couple to, the corresponding aseptic barrier 152 such that both barriers 131, 152 may be removed simultaneously, as described further below.

The hollow needle 129 is mounted within the housing through a collar 132 that is spring-biased in this particular example by a first helical spring 133a and a second helical spring 133b. In other embodiments, the hollow needle 129 may be mounted in another suitable manner, for example, the hollow needle 129 may be statically mounted, i.e. such that it does not move, and the housing may be moveable about the hollow needle 129. Further alternatively, the hollow needle 129 may be axially moveable, via the collar 132, and the upper housing portion 123a may also be axially movable whilst the lower housing portion 123b remains static, i.e. such that it does not move. To this end, the upper housing portion 123a may be provided with actuatable lugs, and the collar 132 may be provided with actuatable lugs, each set of actuatable lugs being actuatable by an actuation mechanism arranged to move the hollow needle 129 and upper housing portion 123a to cause piercing of respective septum seals 128a, 128b. In the present example, the order of piercing of the septum seals 128a, 128b is controlled by virtue of the respective spring forces of springs 133a, 133b. In particular, the first spring 133a provides a first biasing force to the hollow needle 129, via the collar 132, in a direction towards the proximal end 125b of the housing, and the second spring 133 provides a second biasing force to the hollow needle 129, via the collar 132, in a direction towards the distal end 125a of the housing. The first biasing force may be greater than, equal to, or less than the second biasing force, so as to control sequential piercing of the septum seals 128a, 128b. In this example, the first biasing force is greater than the second biasing force, by virtue of a larger wire diameter of first spring 128a compared to second spring 128b.

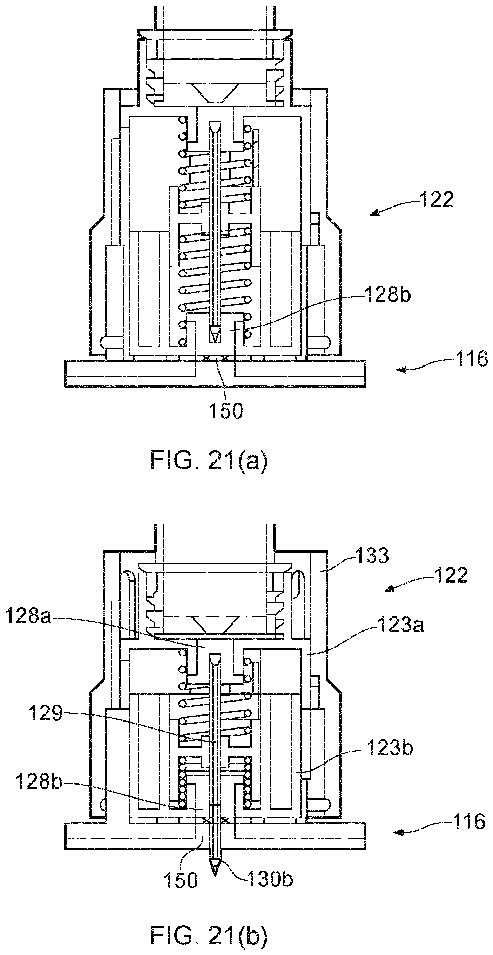

In use, as would be recognised by a person skilled in the art, the hollow needle 129 is caused to engage, and pierce through, respective ports 128a, 128b to fluidly couple a receptacle 124 (see Figure 12(a)) to a second volume of fluid, such as container 118 connected to a planar interface 116, 216, 216a (see, for example, Figures 3, 5 and 6). In order to achieve such piercing, the connector 122 is provided with an actuation mechanism, provided as an outer cover 133, enclosing the upper and lower housing portions 123a, 123b, in the present example. The outer cover 133 is rotatable about the central longitudinal axis of the connector 122. The outer cover 133 includes a protrusion (not shown) on its inner surface which engages with a slot (not shown) on the outer surface of the upper housing portion 123a. The protrusion and slot provide a cam mechanism, such that rotation of the outer cover 133 causes axial translation of the upper housing portion 123a with respect to the lower housing portion 123b. Generally, in use, the second septum seal 128b is arranged to cooperate with a port, such as a septum seal, of a planar interface, as described below. To this end, the hollow needle 129, during piercing, may pierce through both the second septum seal 128b and the septum seal of the planar interface, to provide a fluid communication pathway through such septum seals. The method of fluid connection is provided in more detail with reference to Figures 21(a) to 21(d) below.

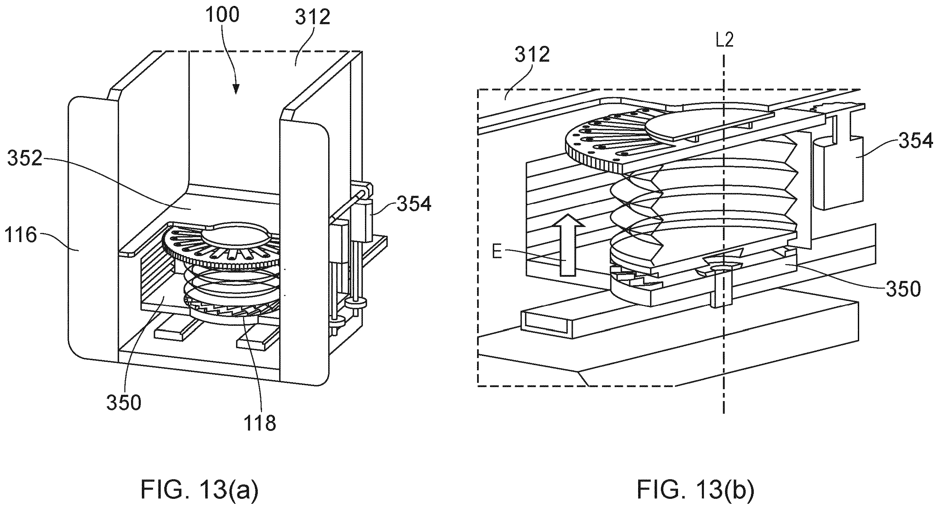

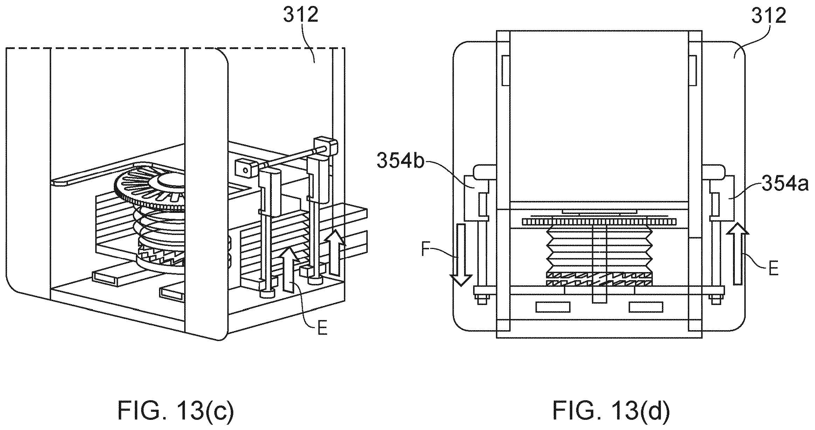

Figures 13(a) to 13(d) illustrate another view of the assembly 100 and enclosure 312 of Figure 11. In particular, a number of linear actuators 354 are provided as part of the enclosure 312, arranged to move the agitation plate 350, in use. As shown in Figures 13(b) and 13(c), the linear actuators 352 are configured to move the agitation plate 350 along a longitudinal axis L2, in the direction of arrow E. There may be four linear actuators 354, specifically a pair of linear actuators 354a, 354b disposed on opposing sides of the assembly 100, particularly at opposing sides of the planar interface 116 and container 118. The linear actuators 354a, 354b are arranged and configured to act synchronously. For example, a pair of linear actuators 354a acting in one direction and the other pair of linear actuators 354b acting in the same direction. In this way, the linear actuators 354a, 354b are arranged to cause linear compression, i.e. axial translation, of the container 118 during use. Such a motion may be useful for, *inter alia,* removing material from the container 118 and/or mixing material within the container 118. Furthermore, as best shown in Figure 13(d), the opposing pair of linear actuators 354a, 354b are arranged and configured to act asynchronously, for example, a pair of linear actuators 354a acting in one direction and the other pair of linear actuators 354b acting in the opposing direction, thereby imparting a rocking, or wave, motion to the container 118, as illustrated by arrows E and F in Figure 13(d). Such a motion may be useful for, *inter alia,* mixing material within the container 118, such as resuspending biological material, such as cells, within a medium therein.

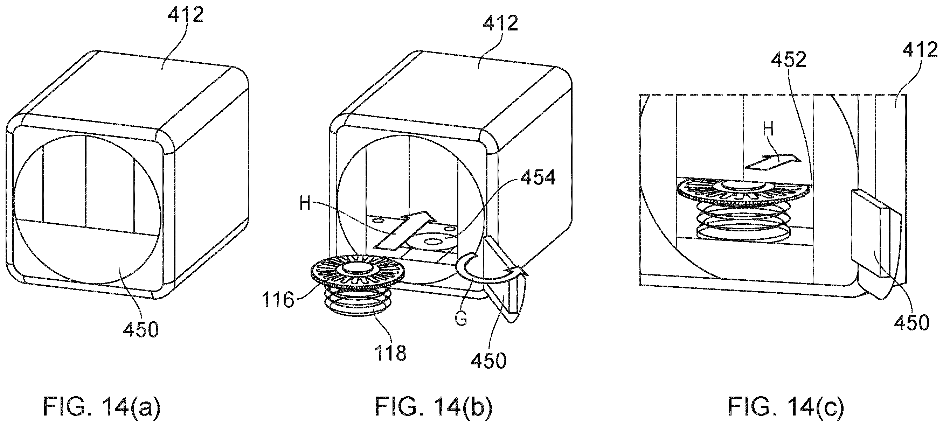

Figures 14(a) to 14(c) illustrate another enclosure 412 for use with the assembly as described herein. In particular, as shown in Figures 14(a) and 14(b), the enclosure 412 includes a hinged door 450, arranged to rotate in a direction G about an axis between a closed configuration (Figure 14(a)), enclosing the contents of the enclosure 412, and an open position (Figure 14(b)), allowing the contents of the enclosure 412 to be accessed. In this particular example, the planar interface 116 and container 118 are slidable into the enclosure 412 (Figures 14(b) and 14(c)) as illustrated by arrow H, the circumferential rim of the planar interface 116 being slidably received within a slot 452 of a plate (see, for example, plate 352 of Figure 13(a)) of the enclosure 412. Furthermore, locating features 454 may be provided in an agitation plate of the enclosure 412 to aid loading of the planar interface 116, particularly the container 118 attached thereto, into the enclosure 412.

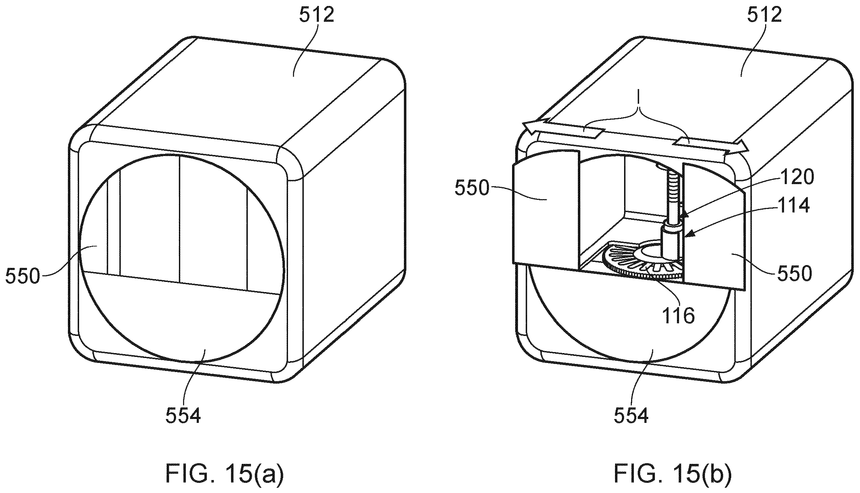

Figures 15(a) and 15(b) illustrate another enclosure 512 for use with the assembly as described herein. In particular, the enclosure 512 comprises a pair of slidable doors 550, moveable within a plane of said doors 550 away from one another. Thus, the slidable doors 550 may be moveable between a closed position (Figure 15(a)), in which the contents of the enclosure and enclosed, and an open position (Figure 15(b)), in which the contents of the enclosure 512 can be accessed. In this particular example, the doors 550 are openable in a direction I so as to allow access to the component retaining element 114, the component 120 and/or the upper surface of the planar interface 116. In this way, the upper components of the enclosure 512, i.e. the component retaining element 114, the upper surface of the planar interface 116, and the component 120, are accessible by virtue of doors 550, whilst the lower components of the enclosure 512, i.e. the container (not shown) and the lower part of the planar interface 116, are accessible by virtue of a drawer or hinged door 554, as described in the above examples. Thus, various accessible parts are compartmentalised, and thus user access can be limited to certain parts of the system during use.

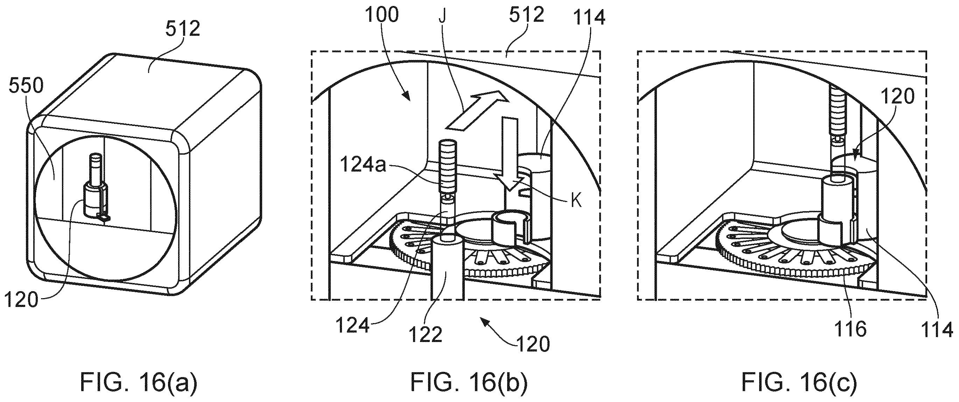

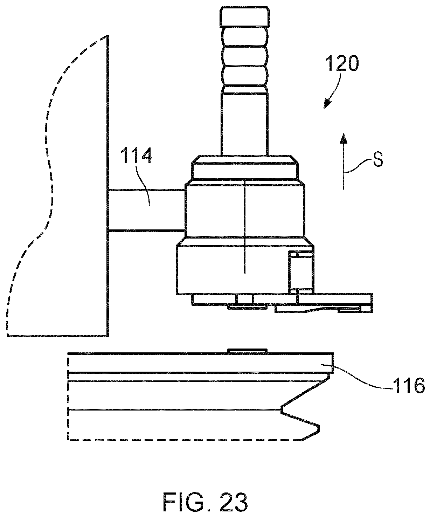

The use of the assembly, and its components thereof, as well as the exemplary enclosures shall now be described by way of one non-limiting example and with reference to Figures 16(a) through to Figure 23.

Referring firstly to Figure 16(a), the component 120 to be retained by the component retaining element is provided, as is an enclosure 512, as described in relation to Figures 15(a) and 15(b), though such an enclosure may be any of the enclosures described herein. The enclosure 512, particularly the upper slidable doors 550 thereof, are opened (Figure 16(b), thereby revealing the component retaining element 114 of the assembly 100 within the enclosure 512. The component 120, shown as a connector 122 having a receptacle 124 including a compressible plunger 124a, is placed within the component retaining element 114, specifically within the component retaining head, as indicated by arrows J and K, and retained by the same (Figure 16(c)). The component retaining element 114 may retain the component 120 by any appropriate means, as described above, but the component retaining element 114 retains the component 120 in this example by virtue of a rail formed within the component retaining head cooperating with a portion, such as a protrusion, of the component 120. In this particular example, the planar interface 116 and the container (not shown) are pre-loaded within the enclosure 512. As will be appreciated by the person skilled in the art, the planar interface 116 and container (not shown) may be loaded in accordance with any example described above.

The user may close the slidable doors 550 so as to enclose the contents of the enclosure 512 following loading.

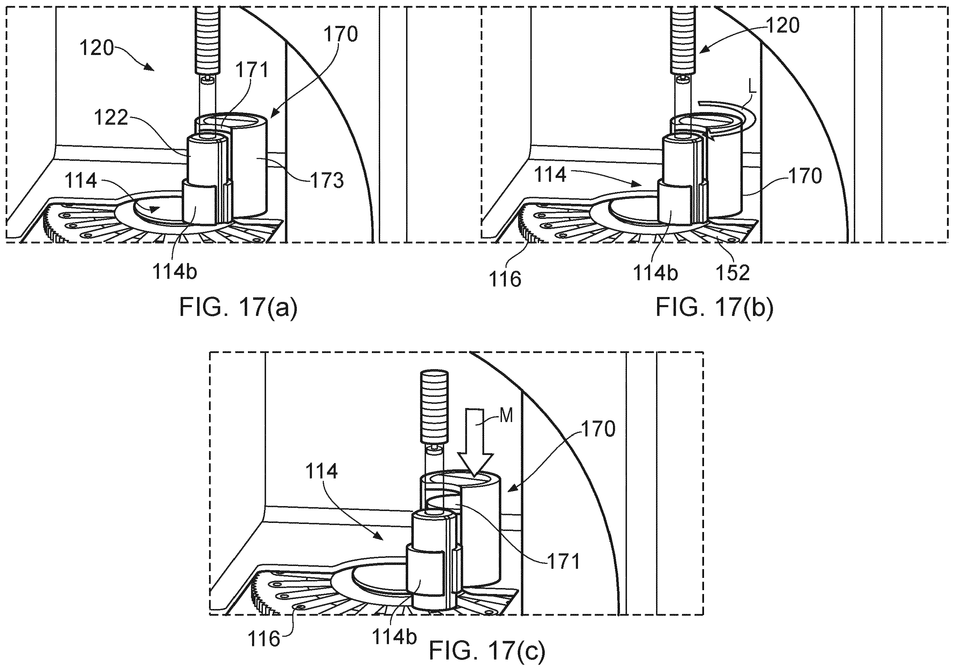

Referring to Figures 17(a) to 17(c), a method of engaging a component, retained by a component retaining element, with a planar interface is illustrated. As shown in Figure 17(a), once the component 120 is loaded to the component retaining element 114, a controller activates the actuator 170. To this end, the user may provide input to a user interface of the controller. In particular, the actuator 170, formed as part of the component retaining element 114, includes a rotatable disc 171, having a cut-out portion therein, housed within an actuator housing 173. The housing 173 similarly has a cut-out portion. The respective cut-out portions are arcuate so as to provide a half-moon shaped cut-out. Thus, as shown in Figure 17(a), with the respective cut-out portions aligned, the component 120 is allowed to be loaded to the component retaining head 114b prior to use. Referring further to Figure 17(b), upon activation, the rotatable disc 171 rotates about a central longitudinal axis of the component retaining head 114b, in the direction L indicated, so as to expose an engagement surface of the disc 171 that is arranged to engage the component 120. The engagement surface in this example is provided as a segment of the rotatable disc 171. The rotatable disc 171 is also moveable axially, as shown in Figure 17(c), such that the engagement surface engages with an upper surface of the component 120, thereby axially moving the component 120 within the component retaining head 114b, due to the cooperating rail configuration of the component retaining head 114b and the component 120. To this end, the component 120 is slidably received, and thus may be slidable or axially moveable, within the component retaining head 114b. The component 120 is axially moved in direction M indicated, until an aseptic barrier 131 of the connector 122 of the component 120 (see Figure 12(b)) engages with a corresponding aseptic barrier 152 of the planar interface 116. In particular, the aseptic barriers 131, 152 are caused to engage in a face-to-face manner.

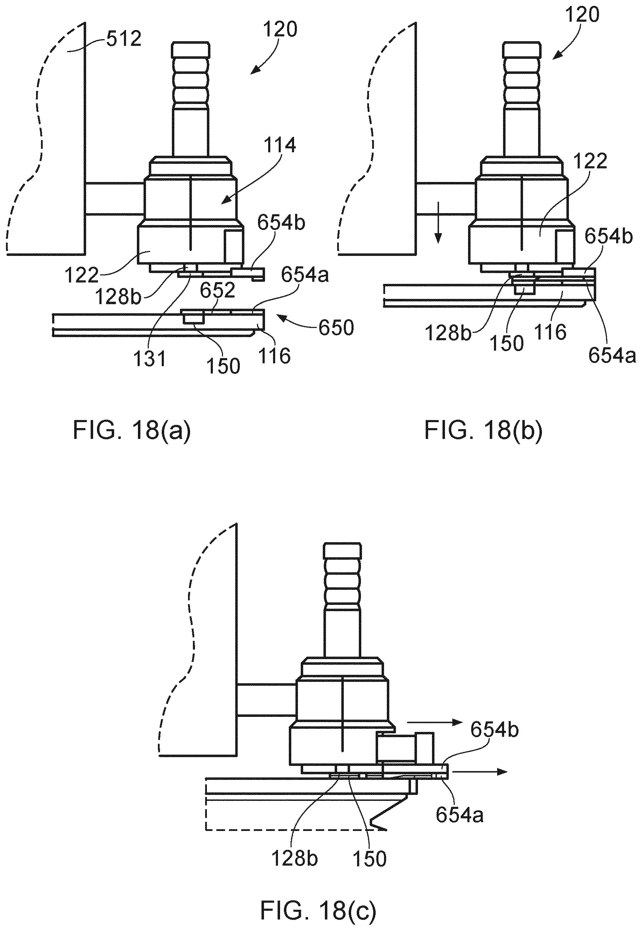

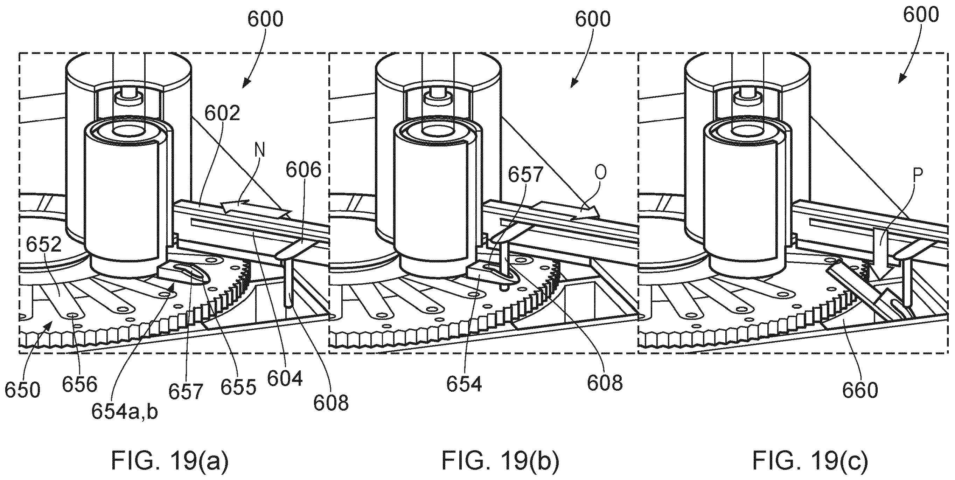

Referring to Figures 18(a) to 18(c), a schematic of one example of the component retaining element 114, the component 120, and the planar interface 116 housed within the enclosure 512 is illustrated. In this example, another example of an aseptic barrier 650 is provided over the port 150 of the planar interface 116. The aseptic barrier 650 includes an aseptic skin 652, disposed over the port 150, and terminates in a fastener portion 654a. The fastener portion 654a is generally received within a slot (not shown) of the planar interface 116, such that the fastener portion 654a is slidable within the slot between a first configuration, in which the aseptic skin 652 is disposed over the port 150, and a second configuration, in which the aseptic skin 652 is removed from the port 150. The fastener portion 654a is generally composed of rigid plastics material, whilst the aseptic skin 652 is composed of a paper or polymer material. The fastener portion 654a is coupled to the aseptic skin 652 in any appropriate manner, for example, through mechanical coupling of a protrusion to an aperture (see Figures 19(a) through 19(c)) of the aseptic skin 652, through adherence to the aseptic skin 652, or the like.

Moreover, in this particular example, an aseptic barrier 131 of the connector 122 (see also Figure 12(b)), disposed over the port 128b of the connector 122, further includes an aseptic skin coupled to a fastener portion 654b. However, in other examples and as will be appreciated by the person skilled in the art, the fastener portions 654a, 654b may be provided on only one of the component 120 or the interface plate 116. In this example, the fastener portions 654a, 654b have corresponding features, such as a protrusion and aperture arrangement, such that the fastener portions 654a, 654b are arranged to couple to one another in use. Referring further to Figures 18(b) and 18(c), and as described further below, the fastener portions 654a, 654b couple to one another, and their respective aseptic skins also couple to one another, upon face-to-face engagement of the connector 122 with the planar interface 116 (see Figure 18(b)). Thereafter, the aseptic barriers 131, 650 are removed, primarily through removal of the coupled fastener portions 654a, 654b, from the component 120 and the planar interface 116 respectively (see Figure 18(c)). In this way, the lateral movement the fastener portions 654a, 654b, connected to their respective aseptic skins, causes the aseptic skins to be removed from their respective ports 150, 128b, thus allowing the ports 150, 128b to engage one another in a face-to-face engagement.

Referring to Figures 19(a) to 19(c), an aseptic barrier removal system 600 is illustrated, and a method of operation thereof shown. The aseptic barrier removal system 600 includes a laterally extending beam 602, including a rail 604 in a side wall thereof. There is also provided a slidable arm member 606, slidably received within the rail 604 and slidable along and within the rail 604, terminating in a longitudinally extending actuation protrusion 608.

In the present example, the aseptic barriers 131, 650 of Figures 18(a) to 18(c) are provided for use with the aseptic barrier removal system 600. In use, the aseptic barrier removal system 600 is arranged to remove the aseptic barriers 131, 650 of the connector 120 and the planar interface 116 (see Figure 18(a)), which are cooperatively coupled together as noted above. In particular, in the present example, the actuation protrusion 608 is moved in a direction N along the rail 604 such that it engages the coupled fastener portions 654a, 654b, as shown in Figure 19(a). More particularly, the fastener portions 654a, 654b may have a lead-in edge 655 (see Figure 19(a)) to guide the protrusion 608 into an aperture 657 (see Figure 19(b)) of the fastener portions 654a, 654b. Upon engagement between the actuation protrusion 608 and the fastener portions 654a, 654b, the actuation protrusion 608 is caused to retract, i.e. move in a direction O along the rail 604 away from the component 120, such that it applies a removal force to the fastener portions 654a, 654b. In this way, the fastener portions 654a, 654b are pulled outwardly, away from the component 120 and planar interface 116 respectively, thereby causing the respective aseptic skins to similarly become pulled outwardly and removed from their respective ports 128b, 150 (see Figure 18(a)). In this way, a port 150 of the planar interface 116 and a port, i.e. second septum seal 128b, of the connector 122 are allowed to engage in a face-to-face manner (see Figure 18(c)). Moreover, as the aseptic barriers 131, 650 are removed, they fall into a waste chute 660, as indicated by arrow P in Figure 19(c). The waste chute 660 may provide a passageway, for example to a bin, for removed aseptic barriers directly, or indirectly, into a clinical waste workstream.

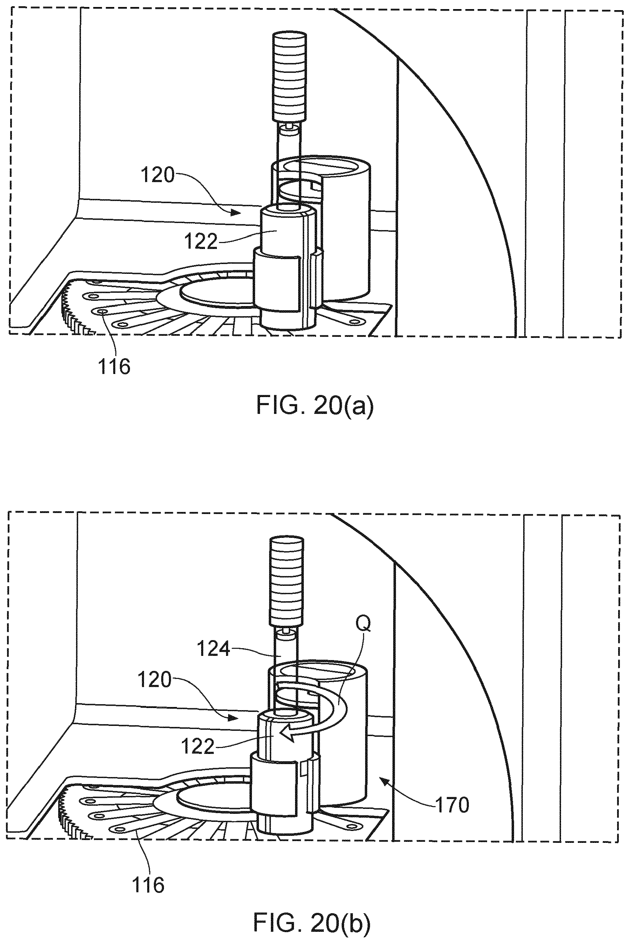

Referring to Figures 20(a) and 20(b), and further to Figures 21(a) to 21(d), a method of actuating the component is described, so as to provide fluid communication through the planar interface.

As shown in Figure 20(a), prior to fluid communication being provided, the component 120, specifically a port thereof, has been brought into register with the planar interface 116, specifically a port thereof. As shown in Figure 20(b), the actuator 170 is arranged to actuate the component 120, specifically the connector 122, to provide fluid communication through the respective ports of the component 120 and the planar interface 116. More specification, the actuator 170 is arranged to rotate, in direction Q about a longitudinal axis of the component 120, specifically the connector 122, as described below. To this end, the actuator 170 may include a pair of gripping fingers (not shown) to enable rotation of the connector 122. In this way, fluid communication is provided between the component 120, specifically the receptacle 124 thereof, and the receptacle (not shown) attached to the planar interface 116.

Referring to Figures 21(a) to 21(d), the various steps of actuation of the component are described. Figure 21(a) illustrates a port, i.e. the second septum seal 128b of the connector 122, of the component 120 brought into register with the port 150 of the planar interface 116. That is to say, the ports 128b, 150 are coaxially, or otherwise, aligned. Figure 21(b) illustrates a first step in the actuation of the connector 122, specifically an initial rotation of the outer cover 133 by the actuator of the component retaining element (not shown). Following such first step in the actuation of the connector 122, the second end 130b of the hollow needle 129 is caused to pierce through the second septum seal 128b of the connector 122, and through the septum seal 150 of the planar interface 116. This is achieved through rotation of the outer cover 133, which collapses the upper housing 123a with respect to the lower housing 123b, thereby causing the hollow needle 129 to pierce the second septum seal 128b and the port 150 at its second end 130b. As illustrated, the opposing first septum seal 128a of the connector 129 remains unpierced due to the spring biasing discussed in relation to Figure 12(b).

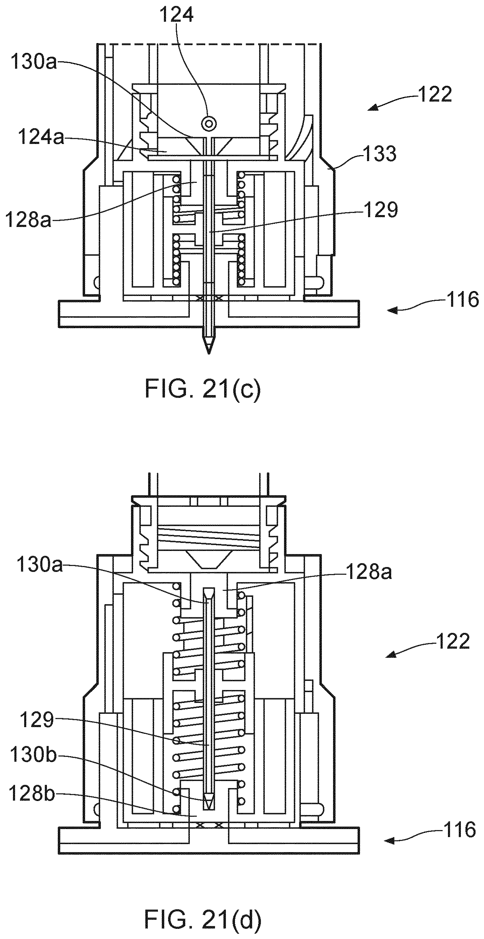

Figure 21(c) illustrates a second step in the actuation of the connector 122, specifically further rotation of the outer cover 133 by the actuator of the component retaining element (not shown). Following such second step in the actuation of the connector 122, i.e. by further rotation of the outer cover 133, the first end 130a of the hollow needle 129 is caused to pierce through the first septum seal 128a of the connector 122, and into the receptacle 124. In this particular example, the receptacle 124 also includes a septum seal 124a, having a trapezoidal cut-out potion, through which the first end 130a of the hollow needle 129 is also caused to pierce through. As shown in Figure 21(c), a fluid communication, through the bore of the hollow needle 129, is provided between the receptacle 124 and the inner volume, for example an attached receptacle, of the planar interface 116. At this point, fluid, such as media, biological material or the like, may be passed between the receptacle 124 and a container attached to the planar interface 116 in either direction. Once fluid communication is desired to be ceased, the outer cover 133 is rotated, by virtue of the actuator of the component retaining element (not shown), in a direction contrary to the direction of rotation to cause piercing of the hollow needle 129 through respective septum seals. Thus, as shown in Figure 21(d), the piercing of each septum seal 128a, 128b is caused to cease as the hollow needle 129 reverts to its original configuration. In particular, the order of cessation of piercing at each septum seal 128a, 128b is contrary to the order of piercing. In other words, in this particular example, the first end 130a of the hollow needle 129 is caused to firstly cease piercing of the first septum seal 128a, and then the second end 130b of the hollow needle is caused to secondly cease piercing of the second septum seal 128b. As will be appreciated by the person skilled in the art, the order of piercing, and the cessation of piercing, may be modified by controlling the spring biasing forces and/or by providing an alternative actuation mechanism of the connector 122.

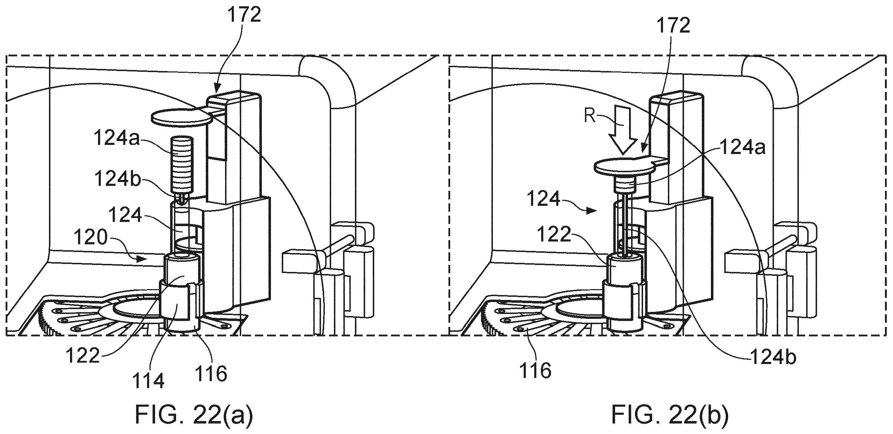

Figures 22(a) and 22(b) illustrate a method of providing fluid dispensation of the receptacle 124 of the component 120 whilst fluid communication is made through the respective ports of the component 120 and the planar interface 116, such as the fluid communication illustrated in Figure 21(c) above. In particular, fluid communication between the receptacle 124 of the component 120, through the planar interface 116, is made to a container (not shown) attached to the planar interface 116, as illustrated in Figures 21(c) and 22(a). Once fluid communication has been established, a dispensing actuator 172, formed as part of the component retaining element 114, acts upon a plunger 124a of the receptacle 124, thereby driving a piston 124b axially downwardly in direction R, i.e. axially toward the connector 122 and the planar interface 116. The dispensing actuator 172 is formed as a paddle driven by a motor in the present example. In other examples, the dispensing actuator 172 may be formed as part of actuator 170 (see Figures 17(a) to 17(c)) discussed above, thereby providing a unitary actuator for actuating the connector 122 and the receptacle 124. As the piston 124b is urged downwardly, the contents of the receptacle 124 are dispensed, through the hollow needle of the connector 122 (see also Figure 21(c)) from the receptacle 124, through the connector 122 and the planar interface 116, and into a container (not shown) attached to the planar interface 116. Thus, an addition of fluid is provided from the component 120 into a container (not shown) attached to the planar interface. Equally, as will be recognised by the person skilled in the art, the dispensing actuator 172 may be replaced by a removal actuator, arranged to act upon the plunger 124a in a direction contrary to that described above, i.e. to move the plunger 124a and the piston 124b axially upwardly, i.e. axially away from the connector 122 and the planar interface 116. Thus, a negative pressure arrangement may be provided, such that contents of a receptacle (not shown) attached to the planar interface 116 are removed, i.e. drawn, into the receptacle 124. Equally, as will be recognised by the person skilled in the art, the actuator 172 may be configured and arranged to both dispense fluid from the receptacle 124 and also draw fluid into the receptacle 124.

Figure 23 illustrates a schematic of a final step in the use of the assembly and other components associated therewith. In particular, once fluid dispensation is complete and the component 120 is disconnected from the planar interface 116, the component retaining element 114, particularly the actuator 170 thereof (see Figure 17(b)), removes the component 120 from the planar interface 116 by axially translating the component 120 in a direction S away from the planar interface 116. The component 120 may then be removed from the component retaining element 114 by a user, or by a robotic arm of the enclosure, or the like, and replaced with another component. The operation of the assembly and like features may then be repeated, as many times as desired, by making use of subsequent port(s) of the planar interface 116. Particularly, the planar interface 116 may be caused to rotate so as to align a port of the new component and subsequent port(s) of the planar interface 116, as illustrated in Figures 7 and 9(c).

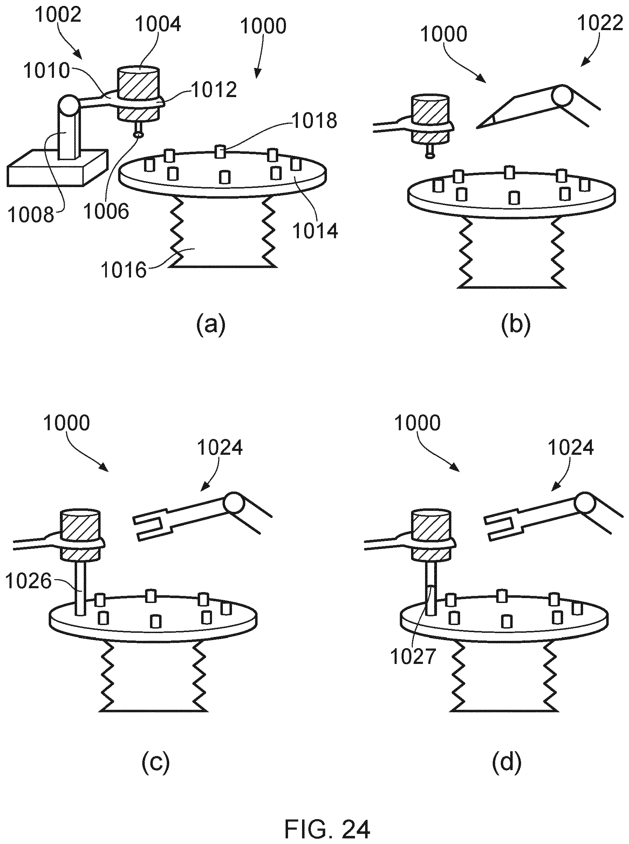

As illustrated in Figures 24(a) through 24(d), another embodiment of an assembly 1000 for handling biological material is disclosed. As illustrated in Figure 24(a), the assembly 1000 includes a component retaining element 1002 that retains a container 1004 having a tube 1006. The tube 1006 may include an aseptic membrane (not shown) coupled to its distal end. The component retaining element 1002 is formed as a robotic arm 1008 terminating in a retaining portion 1010 formed by gripping arms 1012. The gripping arms 1012 are configured to grip and retain the container 1004 during use. The robotic arm 1008 is moveable to move the container 1004 during use. The assembly 1000 also includes a substantially planar interface 1014, such as that described herein, for example in relation to Figures 3 to 7, coupled to a bioreactor 1016. However, in this particular embodiment, the substantially planar interface 1014 includes a plurality of tubes 1018 having aseptic membranes (not shown) coupled to their ends.

As illustrated in Figures 24(b) and 24(c), the assembly 1000 also includes an aseptic tube welder 1022 and an aseptic tube sealer 1024. The aseptic tube welder 1022 is formed at a terminating end of a robotic arm. Similarly, the aseptic tube sealer 1024 is formed at a terminating end of another robotic arm. As such, the respective robotic arms are movable to allow the aseptic tube welder 1022 and aseptic tube sealer 1024 to operate during use. The robotic arms may also cause actuation of their respective aseptic tube welder or sealer 1022, 1024.

The assembly 1000 will now be described in use with respect to Figures 24(a) to 24(d). As shown in Figures 24(a) and (b), the component retaining element 1002 is moveable so as to bring the tube 1006 of the container 1004 into registration with a tube 1018 of the planar interface 1014. Once the tubes 1006, 1018 are brought into registration with one another, as shown in Figure 24(b), the aseptic tube welder 1022 is actuated so as to form an aseptic weld between the respective tubes 1006, 1018, as shown in Figure 24(c). The aseptic membranes, where coupled, may also be removed during this step, either as part of the welding process, i.e. through heat welding, melting or the like, or in a separate removal actuation process operated by the aseptic tube welder 1022 or another actuation component. The aseptic tube welder 1022 thus forms an aseptic weld between the tubes 1006, 1018 and fluidly connects the lumen of each tube 1006, 1018. In this way, an aseptic fluid pathway 1026 is provided through the connected tubes 1006, 1018 to connect the container 1004 and the bioreactor 1016 through the planar interface 1014, as shown in Figure 24(c). Biological material is then transferred from the container 1004 and into the bioreactor 1016, for example, in a manner as described above. As shown in Figure 24(d), once biological material has been transferred, the aseptic tube sealer 1024 is actuated so as to form an aseptic seal between the connected tubing 1006, 1018. In this way, the aseptic fluid pathway 1026 is prevented between the container 1004 and the bioreactor 1016 by virtue of a heat seal 1027. The aseptic tube sealer 1026 may also have a cutting element, arranged to cut the sealed tubing 1006, 1018 along the heat seal 1027 to release the respective components, or the user may provide the necessary cutting in a manual step. Thereafter, the container 1004 is removed from the planar interface 1014. The container 1004 may then be discarded, and the component retaining element 1002 may retain the next container 1004 for the next addition into, or removal from, the bioreactor 1016 via the planar interface 1014, and the process may be repeated starting from Figure 24(a). Generally, those skilled in the art are familiar with aseptic tube welders and sealers, and as such they are not described further herein. The present example provides an automated, or at least semi-automated, manner of aseptically connecting and disconnecting fluid volumes, suitable for "just-in-time" additions or removals of media to, or from, the bioreactor.

## Claims

1. An assembly for handling biological material, comprising:
a first biological handling element, comprising a first volume having a first port;
a second biological handling element, comprising a second volume having a second port;
an aseptic connecting element, configured to aseptically fluidly connect said first port and said second port to provide aseptic fluid communication between said first volume and said second volume;
an aseptic disconnecting element, configured to aseptically fluidly disconnect said first port and said second port to prevent aseptic fluid communication between said first volume and said second volume; and
one or more robotic arms;
wherein the one or more robotic arms are arranged to move said first biological handling element and/or said second biological handling element to bring said first port and said second port into registration with one another to allow, when in use, said aseptic connecting element to aseptically fluidly connect said first port and said second port, and to allow, when in use, said aseptic disconnecting element to aseptically fluidly disconnect said first port and said second port.

2. An assembly according to claim 1, wherein said first biological handling element comprises a substantially planar interface having said first port, said first port fluidly coupled, or configured to be fluidly coupled, to said first volume.

3. An assembly according to claim 1 or claim 2, wherein said second biological handling element comprises a component retaining element retaining a component having said second volume and said second port.

4. An assembly according to any one of claims 1 to 3, wherein said aseptic connecting element and said aseptic disconnecting element are a unitary aseptic connecting and disconnecting element.

5. An assembly according to claim 4, wherein said unitary aseptic connecting and disconnecting element is an aseptic connector.

6. An assembly according to any one of claims 1 to 4, wherein said first port comprises a first tube, said second port comprises a second tube, and said aseptic connecting element comprises an aseptic tube welder.

7. An assembly according to any one of claims 1 to 4, or claim 6, wherein said first port comprises said first tube, said second port comprises a second tube, and said aseptic disconnecting element comprises an aseptic tube sealer.

8. An assembly according to any preceding claim, wherein the one or more robotic arms are controlled by a controller.

9. An assembly according to claim 8, wherein the controller comprises a user interface, and wherein the controller is arranged to control the one or more robotic arms in response to user input to the user interface.

10. An assembly according to any preceding claim, wherein the assembly in an automated assembly.

11. A method of handling biological material, comprising:
moving, using one or more robotic arms, at least one of a first biological handling element, comprising a first volume having a first port, and a second biological handling element, comprising a second volume having a second port, to bring said first port and said second port into registration with one another;
aseptically fluidly connecting said first port and said second port, thereby providing fluid communication between said first volume and said second volume;
transferring biological material from said first volume to said second volume, or from said second volume to said first volume; and
aseptically fluidly disconnecting said aseptically fluidly connected first port and second port, thereby preventing fluid communication between said first volume and said second volume.

12. The method according to claim 11, wherein the step of aseptically fluidly connecting said first port and said second port comprises aseptically welding said first port to said second port.

13. The method according to claim 11 or claim 12, wherein the step of aseptically fluidly disconnecting said first port and said second port comprises aseptically sealing said connected first port and second port to provide an aseptically sealed first port and an aseptically sealed second port.

## Patentansprüche

1. Anordnung zum Handhaben von biologischem Material, umfassend:
ein erstes biologisches Handhabungselement, das ein erstes Volumen umfasst, das einen ersten Anschluss aufweist;
ein zweites biologisches Handhabungselement, das ein zweites Volumen umfasst, das einen zweiten Anschluss aufweist;
ein aseptisches Verbindungselement, das dazu konfiguriert ist, den ersten Anschluss und den zweiten Anschluss aseptisch fluidisch zu verbinden, um eine aseptische Fluidkommunikation zwischen dem ersten Volumen und dem zweiten Volumen bereitzustellen;
ein aseptisches Trennungselement, das dazu konfiguriert ist, den ersten Anschluss und den zweiten Anschluss aseptisch fluidisch zu trennen, um eine aseptische Fluidkommunikation zwischen dem ersten Volumen und dem zweiten Volumen zu verhindern; und
einen oder mehrere Roboterarme;
wobei der eine oder die mehreren Roboterarme angeordnet sind, um das erste biologische Handhabungselement und/oder das zweite biologische Handhabungselement zu bewegen, um den ersten Anschluss und den zweiten Anschluss in Ausrichtung miteinander zu bringen, um zu ermöglichen, dass das aseptische Verbindungselement, wenn es verwendet wird, den ersten Anschluss und den zweiten Anschluss aseptisch fluidisch verbindet, und um zu ermöglichen, dass das aseptische Trennungselement, wenn es verwendet wird, den ersten Anschluss und den zweiten Anschluss aseptisch fluidisch trennt.

2. Anordnung nach Anspruch 1, wobei das erste biologische Handhabungselement eine im Wesentlichen planare Schnittstelle umfasst, die den ersten Anschluss aufweist, wobei der erste Anschluss fluidisch an das erste Volumen gekoppelt oder dazu konfiguriert, fluidisch an dieses gekoppelt zu werden.

3. Anordnung nach Anspruch 1 oder Anspruch 2, wobei das zweite biologische Handhabungselement ein Komponentenrückhalteelement umfasst, das eine Komponente zurückhält, die das zweite Volumen und den zweiten Anschluss aufweist.

4. Anordnung nach einem der Ansprüche 1 bis 3, wobei das aseptische Verbindungselement und das aseptische Trennungselement ein einheitliches aseptisches Verbindungs- und Trennungselement sind.

5. Anordnung nach Anspruch 4, wobei das einheitliche aseptische Verbindungs- und Trennungselement ein aseptischer Verbinder ist.

6. Anordnung nach einem der Ansprüche 1 bis 4, wobei der erste Anschluss ein erstes Rohr umfasst, der zweite Anschluss ein zweites Rohr umfasst und das aseptische Verbindungselement eine aseptische Rohrschweißvorrichtung umfasst.

7. Anordnung nach einem der Ansprüche 1 bis 4 oder Anspruch 6, wobei der erste Anschluss das erste Rohr umfasst, der zweite Anschluss ein zweites Rohr umfasst und das aseptische Trennungselement eine aseptische Rohrversiegelungsvorrichtung umfasst.

8. Anordnung nach einem vorhergehenden Anspruch, wobei der eine oder die mehreren Roboterarme durch eine Steuerung gesteuert werden.

9. Anordnung nach Anspruch 8, wobei die Steuerung eine Benutzerschnittstelle umfasst und wobei die Steuerung angeordnet ist, um als Reaktion auf eine Benutzereingabe an der Benutzerschnittstelle den einen oder die mehreren Roboterarme zu steuern.

10. Anordnung nach einem vorhergehenden Anspruch, wobei die Anordnung eine automatisierte Anordnung ist.

11. Verfahren zum Handhaben von biologischem Material, umfassend:
Bewegen mindestens eines von einem ersten biologischen Handhabungselement, das ein erstes Volumen umfasst, das einen ersten Anschluss aufweist, und einem zweiten biologischen Handhabungselement, das ein zweites Volumen umfasst, das einen zweiten Anschluss aufweist, unter Verwendung eines oder mehrerer Roboterarme, um den ersten Anschluss und den zweiten Anschluss in Ausrichtung miteinander zu bringen;
aseptisches fluidisches Verbinden des ersten Anschlusses und des zweiten Anschlusses, wodurch eine Fluidkommunikation zwischen dem ersten Volumen und dem zweiten Volumen bereitgestellt wird;
Übertragen von biologischem Material von dem ersten Volumen zu dem zweiten Volumen oder von dem zweiten Volumen zu dem ersten Volumen; und
aseptisch fluidisches Trennen des aseptisch fluidisch verbundenen ersten Anschlusses und zweiten Anschlusses, wodurch eine Fluidkommunikation zwischen dem ersten Volumen und dem zweiten Volumen verhindert wird.

12. Verfahren nach Anspruch 11, wobei der Schritt des aseptischen fluidischen Verbindens des ersten Anschlusses und des zweiten Anschlusses aseptisches Schweißen des ersten Anschlusses an den zweiten Anschluss umfasst.

13. Verfahren nach Anspruch 11 oder Anspruch 12, wobei der Schritt des aseptischen fluidischen Trennens des ersten Anschlusses und des zweiten Anschlusses aseptisches Versiegeln des verbundenen ersten Anschlusses und des zweiten Anschlusses umfasst, um einen aseptisch versiegelten ersten Anschluss und einen aseptisch versiegelten zweiten Anschluss bereitzustellen.

## Revendications

1. Ensemble destiné à la manipulation d'un matériel biologique, comprenant :
un premier élément de manipulation biologique, comprenant un premier volume comportant un premier orifice ;
un second élément de manipulation biologique, comprenant un second volume comportant un second orifice ;
un élément de raccordement aseptique, conçu pour raccorder de manière aseptique et fluide ledit premier orifice et ledit second orifice de manière à assurer une communication fluidique aseptique entre ledit premier volume et ledit second volume ;
un élément de découplage aseptique, conçu pour découpler de manière aseptique et fluide ledit premier orifice et ledit second orifice afin d' empêcher une communication fluidique aseptique entre ledit premier volume et ledit second volume ; et
un ou plusieurs bras robotiques ;
lesdits un ou plusieurs bras robotiques étant agencés de manière déplacer ledit premier élément de manipulation biologique et/ou ledit second élément de manipulation biologique afin d'amener ledit premier orifice et ledit second orifice en alignement l'un avec l'autre pour permettre, lors de l'utilisation, audit élément de raccordement aseptique de raccorder de manière aseptique et fluide ledit premier orifice et ledit second orifice, et pour permettre, lors de l'utilisation, audit élément de découplage aseptique de découpler de manière aseptique et fluide ledit premier orifice et ledit second orifice.

2. Ensemble selon la revendication 1, ledit premier élément de manipulation biologique comprenant une interface sensiblement plane comportant ledit premier orifice, ledit premier orifice étant couplé de manière fluidique, ou conçu pour être couplé de manière fluidique, audit premier volume.

3. Ensemble selon la revendication 1 ou la revendication 2, ledit second élément de manipulation biologique comprenant un élément de retenue de composant retenant un composant comportant ledit second volume et ledit second orifice.

4. Ensemble selon l'une quelconque des revendications 1 à 3, ledit élément de raccordement aseptique et ledit élément de découplage aseptique étant un élément de raccordement et de découplage aseptique unitaire.

5. Ensemble selon la revendication 4, ledit élément unitaire de raccordement et de découplage aseptique unitaire étant un raccord aseptique.

6. Ensemble selon l'une quelconque des revendications 1 à 4, ledit premier orifice comprenant un premier tube, ledit second orifice comprenant un second tube, et ledit élément de raccordement aseptique comprenant un poste de soudure de tube aseptique.

7. Ensemble selon l'une quelconque des revendications 1 à 4, ou la revendication 6, ledit premier orifice comprenant ledit premier tube, ledit second orifice comprenant un second tube, et ledit élément de découplage aseptique comprenant une pince à sceller les tubes aseptique.

8. Ensemble selon une quelconque revendication précédente, lesdits un ou plusieurs bras robotiques étant commandés par un dispositif de commande.

9. Ensemble selon la revendication 8, ledit dispositif de commande comprenant une interface utilisateur, et ledit dispositif de commande étant agencé de manière à commander lesdits un ou plusieurs bras robotiques en réponse à une entrée utilisateur sur l'interface utilisateur.

10. Ensemble selon une quelconque revendication précédente, ledit ensemble étant un ensemble automatisé.

11. Procédé de manipulation d'un matériel biologique, comprenant :
le déplacement, à l'aide d'un ou plusieurs bras robotiques, d'au moins l'un d'un premier élément de manipulation biologique, comprenant un premier volume comportant un premier orifice, et d'un second élément de manipulation biologique, comprenant un second volume comportant un second orifice, pour amener ledit premier orifice et ledit second orifice en alignement l'un avec l'autre ;
le raccordement de manière aseptique et fluide dudit premier orifice et dudit second orifice, assurant ainsi une communication fluidique entre ledit premier volume et ledit second volume ;
le transfert du matériel biologique dudit premier volume audit second volume, ou dudit second volume audit premier volume ; et
le découplage de manière aseptique et fluide dudit premier orifice et dudit second orifice raccordés de manière aseptique et fluide, empêchant ainsi une communication fluidique entre ledit premier volume et ledit second volume.

12. Procédé selon la revendication 11, ladite étape de raccordement de manière aseptique et fluide dudit premier orifice et dudit second orifice comprenant le soudage de manière aseptique dudit premier orifice audit second orifice.

13. Procédé selon la revendication 11 ou la revendication 12, ladite étape de découplage de manière aseptique et fluide dudit premier orifice et dudit second orifice comprenant le scellage de manière aseptique dudit premier orifice et dudit second orifice raccordés de manière à fournir un premier orifice scellé de manière aseptique et un second orifice scellé de manière aseptique.
